Europäisches Patentamt

(19) European Patent Office

Office européen des brevets

(11) Publication number : **0 357 625 B1**

# EUROPEAN PATENT SPECIFICATION

(12)

(45) Date of publication of patent specification :
08.01.92 Bulletin 92/02

(51) Int. Cl.⁵ : **G01N 33/53, G01N 33/52, G01N 33/543**

(21) Application number : 88902879.1

(22) Date of filing : 25.03.88

(86) International application number :
PCT/GB88/00229

(87) International publication number :
WO 88/07679 06.10.88 Gazette 88/22

(54) ASSAY APPARATUS AND USE THEREOF.

(30) Priority : 26.03.87 GB 8707299

(43) Date of publication of application :
14.03.90 Bulletin 90/11

(45) Publication of the grant of the patent :
08.01.92 Bulletin 92/02

(84) Designated Contracting States :
AT BE CH DE FR GB IT LI LU NL SE

(56) References cited :
EP-A- 166 933
AU-A- 540 523
US-A- 3 261 668
US-A- 4 052 161
WO 83/03140

(73) Proprietor : THE SECRETARY OF STATE FOR
HEALTH IN HER BRITANNIC MAJESTY'S
GOVERNMENT OF THE UNITED KINGDOM OF
GREAT BRITAIN
AND NORTHERN IRELAND 14 Russell Square
London WC1B 5EP (GB)

(72) Inventor : BUNCE, Roger, Abraham
117 Berberry Close
Bournville Birmingham B30 1TB (GB)
Inventor : GIBBONS, John, Edwin, Charles
40 St Peters Close
Hall Green Birmingham B28 0EF (GB)
Inventor : MATTHEWS, Jayne, Alison
York Cottage Meadow Lane Woodhouses
Yoxall, Staffordshire DE13 8NP (GB)
Inventor : BURN, Catherine, Ann
132 Gravelly Hill
Erdington Birmingham B23 8NP (GB)
Inventor : KRICKA, Larry, Jan
886 Northern Hale Road
Berwyn, Philadelphia, PA 19312 (US)

(74) Representative : Beckham, Robert William et al
Procurement Executive Ministry of Defence
Room 2016 Empress State Building Lillie Road
London SW6 1TR (GB)

Jouve, 18, rue Saint-Denis, 75001 PARIS

## Description

This invention relates to an assay apparatus for use in the detection of an immobilised analyte or an immobilised analyte-bindable substance. The invention further relates to a method of using the apparatus in an assay, particularly in an enzyme immunoassay, and to a kit and to an analytical device incorporating the apparatus.

Analytical devices used to detect and/or quantify immobilised analytes are employed extensively in analytical chemistry. They are particularly useful in detecting and/or quantifying substances of biological interest, **eg antigenic proteins, polypeptides, polynucleotides,** hormones, haptens, antibodies and drugs from body fluids and tissues such as whole blood, serum, body secretions, urine, tissue extract etc. One well known method of analysis in which such devices are used is enzyme immunoassay (EIA). A typical EIA analysis involves the following process steps:-

(1) Specific antibodies are immobilised onto a solid substrate (typically a plastic material).

(2) The immobilised antibodies are brought into contact with a sample solution, eg diluted whole blood or serum, suspected of containing an antigenic analyte. Any analyte contained in the sample is specifically bound by the antibody attached to the solid substrate.

(3) The excess sample is washed off.

(4) The bound analyte is brought into contact with a solubilised reagent consisting of an enzyme-labelled antibody and the antibody and hence the label is attracted to and bound to any analyte bound by the immobilised antibody to the solid support.

(5) The excess reagent is washed off.

(6) The bound enzyme-labelled antibody is brought into contact with a final reagent containing chemicals which react with any bound enzyme label present to give an output signal(for example an output of light),either directly or as a result of external stimulation, which is dependent upon the amount of analyte in the sample. The signal is measured using an appropriate instrument; for example, when the reaction between enzyme and final reagent consists of a chemi- or bio-luminescent reaction, the output of light produced may be measured with a luminometer. Many other analytical processes involving immobilised analytes have a similar number of process steps.

The solid substrate which is often made of plastic and provided in the form of a reactive strip or stick, has hitherto been consecutively transferred between a series of open-topped vessels during these process steps, the vessels containing in turn sample solution,washing solutions, and reagent solutions. The many steps that are involved in an analytical procedure of the type exemplified by EIA analysis in particular therefore gives rise to a procedure which frequently involves the use of relatively large quantities of sample solution and reagent, and is moreover complex and costly to automate. Where body fluids such as blood serum is being analysed, this may involve taking several ml of fresh blood, a traumatic experience for some donors, in order to collect sufficient sample solution to perform the analysis.

A further disadvantage of typical EIA analytical procedures in particular is that it is difficult to prevent contamination of the sensitised area of the solid substrate to which analyte is bound whilst the analysis is being carried out in view of the many process steps that are involved and the frequent exposure of the sensitised area to the atmosphere. Of even greater concern in the analysis of body fluids and tissues are the possible dangers to operators of typical analytical procedures caused by their exposure to harmful materials in the sample being analysed during the often many manual transfer steps involved. For example where blood or serum is being analysed it could be contaminated with HTLV III (AIDS virus) or hepatitis virus. Where attempts have been made to automate analytical procedures such as those involved in a typical EIA, these have frequently involved costly and complex analytical systems which are very difficult to decontaminate.

It is one object of the present invention to overcome at least some of these disadvantages and to provide a relatively simple, inexpensive, and safe means of analysis by successive liquid transfer and excess liquid removal steps.

According to a first aspect of the present invention,there is provided an assay apparatus for use in the detection and/or quantitative measurement of an analyte immobilised on an active surface portion of a solid substrate, comprising a substrate housing means for retaining the solid substrate at one or more predetermined positions, first reagent transfer means for transferring a solubilised first reagent to the surface portion when the substrate is held at one of the predetermined positions thereby coating the surface portion with solubilised reagent, the reagent being selected to react with either the analyte or the surface portion and emit a signal, either directly or in the present of a signal stimulant, providing a measure of the amount of the analyte immobilised on the surface portion, and substrate wiping means, the solid substrate and wiping means being moveable relative to each other when the substrate is wholly or partly retained within the housing means said wiping means being arranged to make intimate wiping contact with all or part of the surface portion before and/or after transfer of solubilised first reagent thereto by the first reagent transfer means to remove excess liquid therefrom.

The term "active surface portion" as used in this specification means a surface portion which is prepared or treated to entrap a specified analyte thereon, either by chemical or physical interaction between the surface portion and analyte.

The main advantage of an assay apparatus in which both liquid transfer and excess liquid removal can be performed consecutively within a housing means is that it both simplifies the performance of these steps and obviates the need for these steps to be performed in separate containers which have hitherto usually involved exposing the sensitised surface of the substrate to the environment. Furthermore, the inventors' discovery that mechanical wiping rather than washing of the substrate is an efficient and effective method of removing unwanted solubilised reagent not only means that the number of separate vessels required for washing the substrate is correspondingly reduced, but also that the reagent removal step is much simpler to perform and (if necessary) to automate.

The present apparatus preferably includes at least one wiping means arranged to wipe the surface portion after transfer of first reagent in order to prevent the presence of unreacted excess first reagent from interfering with the size of the emitted signal.

The present apparatus may be used in two different ways, either in a competitive or non-competitive assay for analyte present in a sample solution.

In a competitive assay, in which both analyte and first reagent compete for reaction with the surface portion of the solid substrate for example a competitive immunoassay, the sample solution may be added before, after, or preferably during transfer of first reagent.

In a non-competitive assay, for example chemical analysis or a non-competitive immunoassay, in which the first reagent is selected to react with the analyte but not the surface portion of the solid substrate underlying the immobilised analyte, analyte will already have been immobilised on the surface position of the solid substrate before transfer of first reagent. This may be achieved by transferring the sample to the surface portion prior to insertion into the housing means, for example by dipping the substrate into the sample, followed preferably by washing and/or wiping the exposed substrate plus bound analylte.

Alternatively and preferably for both competitive and non-competitive assays the apparatus may include sample transfer means arranged to transfer sample solution to the surface portion of the substrate within the housing means, thereby coating the surface portion with sample solution which may be before, during or after transfer of the first reagent. The preferred relative dispositions of wiping, sample and first reagent transfer means may vary depending upon whether the apparatus is intended for competitive or non competitive assays.

For a competitive assay the sample transfer means may be arranged to transfer sample solution to the surface portion of the substrate within the housing means after, or preferably before or with the transfer of the first reagent, but before the surface portion is subjected to the substrate wiping means.

For non-competitive assays the sample transfer means may be arranged to transfer sample solution to the surface portion of the substrate before transfer of the first reagent.

Whether the apparatus contains sample transfer means or not (eg if the substrate is exposed to the sample prior to insertion into the housing means), the apparatus preferably has substrate wiping means, the solid substrate and wiping means being moveable relative to each other when the substrate is wholly or partly retained within the housing means, said wiping means being arranged to make intimate wipin contact with all or part of the surface portion after the transfer of sample and between the transfer of sample solution and solubilised first reagent.

The wiping means for sample solution and for the first reagent are preferably different in order to prevent recontamination of the substrate surface with sample solution after first reagent has been transferred. The preferred apparatus which includes these further transfer and wiping means will therefore be adapted to provide sequential transfer of sample solution and first reagent to the solid substrate, each transfer step being followed by an appropriate wiping means to remove unbound or unreacted material. Alternatively, where the sample solution is tranferred before the substrate is used in the present apparatus, then the apparatus may include wiping means for the sample solution in the position defined above and without the sample transfer means.

Where the present apparatus includes a sample transfer means, the said means may incorporate a filter means to prevent unwanted components in the sample solution from reaching the solid substrate. For example, the filter means may be used to separate out red blood cells from the whole blood, allowing passage of plasma only. Suitable materials and methods of construction of such a filter will be well known to those skilled in the art.

The signal from the surface portion of the substrate after reaction with the first reagent may be emitted directly, for example it may comprise ionising radiation emitted from a first reagent consisting of or containing a radioactive isotope which reacts with the immobilised analyte by binding to it. Where the signal is emitted in the presence of a signal stimulant, the stimulant may be particulate, electromagnetic radiation, heat, electrical or chemical or a combination thereof, and the apparatus may include means for directing said stimulant onto the surface portion of the substrate in the housing means preferably after application thereto of one or more of the wiping means.

It is preferred that the signal stimulant is or includes the use of chemical stimulation, which may take the form of a second solubilised reagent that takes part in a reaction with the first reagent in which either the reaction or a product thereof emits a detectable signal. This reaction may be a chemi- or bio-luminescent reaction. Where chemical stimulation is employed, the present apparatus preferably includes second reagent transfer means for transferring second solubilised reagent to the surface portion, thereby coating said portion with solubilised second reagent, preferably after said portion has been wiped by the wiping means which wipes the substrate after transfer of the first reagent.

Particulate stimulants include photons, especially optical, uv, and x-ray photons, ionizing particles, and electons which may be absorbed, scattered, or reflected by the product of the reaction between analyte and first reagent or used to excite a surface portion-bound first or second reagent containing or consisting of a fluorescent material, in order to produce an emitted signal. Particulate stimulant directing means may comprise an opening in the housing means or preferably, a first window portion in the housing means of material which is substantially transparent to the incident particles. Electrical stimulation may be provided through electrodes postioned within the housing means, or brought into contact with the substrate. The advantage of providing a means for directing signal stimulant, where required, onto the substrate in the housing means is that the signal is then emitted without having to remove the substrate from the housing means.

The apparatus will therefore preferably also include a means for directing the emitted signal out of the housing means, so that it may then be detected by detection means situated outside the housing means. The emitted signal may be particulate, for example in the form of photons or ionizing particles, acoustic, thermal, chemical(eg a pH change) or electrical in nature. Where the emitted signal consists of emitted particles, the means for directing the emitted signal out of the housing means may comprise an opening in the housing means or a second window portion or a light pipe (eg an optical fibre) in the housing means of a material which is substantially transparent to the emitted particles. The window material may consist of glass or transparent plastic where the emitted signal consist of optical photons, and may include filter means to allow passage of certain wavelengths of light only. The edges of the window may be made reflective to reduce light loss through absorbtion by the housing means. If the emitted signal is electromagnetic radiation, eg light, the housing means may include a reflective portion to direct light through the window. The window, if made of a transparent material may be shaped to act as a lense.

The housing means surrounding the window or windows is preferably substantially opaque to both particulate stimulants and particulate emitted signals.

Where the emitted signal consists of light from a chemi- or bio-luminescent reaction initiated by contacting the surface portion with second reagent from a second reagent transfer means, the emitted signal directing means is preferably disposed so that it is capable of receiving emitted light through a transparent or translucent portion of the substrate while the surface portion is in contact with the second reagent transfer means. Such an arrangement ensures a constant supply of second reagent to the reaction while signal detection is being effected.

The wiping means of the present apparatus may each take the form of one or more resilient wiping blades or, more preferably, one or more absorbent pads in which case "wipin" and derived terms used in relation to removal of liquid from a surface may include contacting the surface with a stationary absorbent pad as well as a moving pad. The inventors have found that absorbent pads, which may be made of, for example, glass fibre, cellulosic material or foamed plastic, can be highly efficient in removing excess liquid and unbound solubilised materials from the surface of the substrate. They have the advantage over wiping blades that they retain absorbed liquid, so preventing leakage of wiped liquid to other parts of the apparatus, and conform more readily to the shape of the surface being wiped. Absorbent pads are also relatively inexpensive and easily disposed of and replaced, so allowing the present apparatus to be reused with fresh uncontaminated pads. Alternatively an absorbent pad may form an inexpensive component of a single-use, disposable labelling apparatus. Each of the one or more wiping pads will normally have a certain degree of resilience, so that it may be resiliently urged against the surface of the substrate to effect more efficient wiping. Each pad may be used dry. Alternatively, and preferably, each may be wetted with solvent, eg distilled water or buffer solution, before use to increase wipin efficiency in which case each wiping means may include means for directing solvent onto the pad, such as an opening in the housing means adjacent the pad through which solvent may be delivered. The absorbent wiping pads may have an activated surface, eg to neutrilise a liquid to be wiped off the surface.

The liquid transfer means may each comprise a vessel, for containing sample solution or reagent as the case may be, into which the sensitised surface portion of the substrate may be dipped when the apparatus is in use. Although such means are effective in transferring liquid to the surface portion, it is difficult to avoid using relatively large amounts of fluid and each dipping operation involves a reversal in the direction of substrate travel which may be difficult to automate.

Alternatively, each transfer means may comprise an opening in the housing means that communicates with the surface portion when the substrate is retained at one of predetermined positions. This permits direct delivery of sample solution or reagent as the case may be to the surface portion from, for example, a micropipette or other delivery means.

One form of such an openning comprises a port communicating with the outside of the housing means, through which a sample or reagent may be added, and communicating with a relatively shallow recess in an inner surface portion of the housing means that comes into contact with the substrate when the latter is in place in the housing means. With such an openning, a liquid sample or reagent may be added through the port whilst the substrate is in place, and may then spread through the cavity formed between the substrate and the walls of the recess by capilliary action, coating the surface of the substrate in the area of the recess.

The nature of the sample or reagent may have a bearing on the dimensions of the port and/or recess, and it has been found that for transfer of a sample of whole blood this type of sample transfer means is not favoured, but if used a fairly wide port is advisable.

However, the inventors have found that direct transfer of sample solution and/or solubilised reagent by such delivery means often leads to uneven coating of the substrate surface due to surface tension effects. They have found that these problems may be overcome by providing the or each liquid transfer means in the form of one or more absorbent transfer pads made from, for example, glass fibre, cellulosic material or foamed plastic. Each transfer means may include a further means, such as an opening in the housing means, adjacent the transfer pad, to direct liquid (which will be sample solution or solubilised reagent as the case may be) to the transfer pad. Pads for transferring reagent may have lyophilised reagent impregnated therein, in which case this liquid may comprise solution to solubilise the reagent.

Such pads may have a laminated construction, each laminate containing lyophilised reagent, because certain reagents are unstable when combined.

Such transfer pads may be permanently mounted in the housing means when the housing means is constructed, or alternatively the housing means may be adopted so that pads may be subsequently fitted prior to use. Each transfer pad may be arranged in the housing means to make direct interference contact with or reside in close proximity with the surface portion of the solid substrate when positioned at at least one of its predetermined positions, and preferably each pad has a surface whose area is capable or covering some or all of the sensitised surface portion even and continuous or semi-continuous contact of liquid with the sensitised surface.

The width of the wiping pad or pads is preferably greater than that of the sample transfer pads, and even better, greater than the width of the substrate.

Width is defined as the dimension perpendicular to the direction of relative motion of the substrate and pads. Where a transfer pad is adapted to make direct contact with the substrate, the pad is preferably resilient as well as absorbent so that it may be resiliently urged against the substrate to release its liquid content.

Quite apart from the advantages of low cost and disposability discussed earlier for absorbent pads, a further advantage of the use of absorbent pads as liquid transfer means in the present apparatus is that they can ensure constant and uniform liquid/solid phase interaction, for relatively long periods if required, without having to utilise large volumes of liquid. A further advantage of transfer pads is that due to surface tension effects, liquid can usually be transferred to the substrate with the apparatus disposed in any orientation. Furthermore any excess liquid will normally be retained in the pad and is unlikely to leak to other parts of the apparatus.

The housing means may be adapted to house the solid substrate in a single fixed position, in which case the wiping means will be moveable in relation to the housing means or, preferably, the housing means will include substrate guide means adapted to guide the substrate along a predetermined path through the housing means such that the surface portion passes the reagent transfer and wiping means in the correct sequence. The advantage of a substrate guide means is that the apparatus need contain no moving parts, since each of the one or more wiping means, each of which preferably consists of one or more resilient absorbent wiping pads, may be statically mounted within the housing means in the path of the surface portion.

The wiping and transfer means are preferably physically separated within the apparatus by sufficient distances to prevent direct transfer of liquid between the means. It is also important to avoid transport of liquids, eg sample or reagent through the apparatus eg between pads by capillary action during use. To reduce or avoid this the housing means may be provided with grooves, cavities etc to enlarge internal spaces in appropriate places and of a size that avoids capilliary transport of liquid. Suitable places and sizes will be apparent to those skilled in the art. Alternatively the relative sizes of internal spaces in the apparatus may be made large enough to avoid capilliary action.

For example, if the sample or reagent transfer means comprises a port in the housing means communicating with a recess in its inner surface as described above, the recess may be surrounded by an annular groove in the said inner surface to prevent capilliary flow of sample or reagent from the vicinity of the recess.

Where one or more wiping and/or transfer pads are used, these pads are preferably mounted within the housing means so that they at least partly overlap with the path of the substrate to provide an interference fit between pad and surface portion, thereby promoting more efficient liquid transfer and substrate wiping. Each pad may be mounted so as to present an inclined face to the path of the substrate, in order to facilitate movement of the substrate past the pad. The apparatus may include wiper disengaging means to permit disengagement of wiper means, especially pads, from the surface of the substrate. The direction of substrate travel within the housing means may then be reversed without the wiping means having to engage the surface portion in both directions so avoiding the problem of recontaminating the surface portion. The pad disengaging means for each wiping pad preferably comprises a resilient biasing means, such as a resilient finger, for biasing the pad away from the substrate, and detent means for releasably holding the pad in the path of the subtrate.

Several embodiments of the invention are possible when the apparatus includes a guide means. In one embodiment, the housing means contains two or more cavities, each cavity containing wiping, sample or reagent transfer means provided that at least one of each means is present in the apparatus, and each cavity having substrate guide means adapted to guide the substrate in said cavity. The depth of each of these cavities should be such that the substrate may be inserted therein to an extent that the surface portion is contacted by the wiping or transfer means therein. In one form of this embodiment the cavities may be joined together in an array, or may be of unitary construction. Each wiping means is preferably provided in the form of an absorbent resilient pad, and preferably wiper disengaging means are provided for each. Where three or more cavities are provided, the array preferably comprises an alternating sequence of wiping means and transfer means. Coating and wiping of the substrate surface portion may then be effected by inserting the substrate into the cavities in the correct sequence.

In a further embodiment, the housing means comprises one or more cavities for receiving the solid substrate, each cavity containing transfer means, wiping means disposed in the opening of each cavity, substrate guide means for guiding the substrate into each cavity past the wiping means such that the wiping means makes wiping contact with the surface portion of the substrate, and, optionally, wiper disengaging means. Coating and wiping of the surface portion is effected by inserting the substrate in the correct sequence into the one or more cavities, at least one of which contains a solubilised first reagent in use, and engaging the surface of the substrate with the wiping means when inserting and/or when withdrawing the substrate. The depth and arrangement of the cavities may be as in the embodiment described immediately above.

In a yet further embodiment, the housing means may be adapted to house a flexible solid substrate in the form of an endless tape, the guide means including drive means for moving the tape past liquid transfer means and wiping means. The liquid transfer and wiping means, preferably in the form of pads mounted within the housing means, may be removable so that they may be replaced after coating and wiping has been effected. The apparatus for containing the flexible endless tape may include cleansing means for removing bound material from the substrate and regenerating the surface portion after coating and wiping has been effected. In this way, the apparatus and tape may be reused. Alternatively, a disposable assay apparatus may be provided which will normally exclude a cleansing means.

In a preferred embodiment however, where the apparatus includes a substrate guide means, the housing means preferably comprises an enclosure having a substrate guide means in the form of a channel therein, the transfer means and wiping means being linearly disposed along the channel in the path of the solid substrate. The channel is preferably shaped so as to receive an elongated solid substrate, in particular a flat strip or stick. The channel may be open at one or both ends to permit insertion of the substrate into and/or withdrawal of the substrate from the housing means. Where the channel is open at one or both ends, the substrate may be advanced through the housing means, and if both ends are open, out of the housing means. The transfer and wiping means are arranged in the channel to effect the sequence of transfer and wiping of the surface portion as the substrate is advanced through the channel, and then emission and detection of the signal.

The enclosure may if necessary also include stimulant and signal directing means. The path of the substrate through the channel preferably describes a substantially straight line, although other geometrics, such as a curved, circular or partly circular path may be used.

A preferred sequence of transfer and wiping steps etc in this last embodiment as the substrate is advanced through the housing means is:

    1. transfer of sample to substrate surface portion

    2. wiping by a first wiping means to remove excess sample

    3. transfer of first reagent to substrate surface portion

    4. wiping by a second wiping means to remove excess first reagent

    5. transfer of second reagent to substrate surface

    6. emission and detection of signal.

Steps 5 and 6 may take place with the substrate in the same predetermined position within the housing means.

The preferred embodiment therefore consequently contains a linear arrangement of sample transfer, reagent transfer, wiping and signal directing means to achieve part or preferably all of this sequence. One or both of Steps 1 and 2 may be performed outside the housing means.

In one form of this preferred embodiment, the channel may contain only one set of transfer and wiping means necessary to carry out the sequence of transfer and wiping in respect of a single surface portion of the solid substrate, eg the preferred sequence of steps 1-6 described above.

Alternatively the channel may contain two or more sets of transfer and wiping means necessary for the sequence of transfer and wiping, arranged side by side across the width of the channel, so as to transfer reagent to and wipe several surface portions of the substrate. With such an arrangement, for example, assays for several different analytes on one sample may be carried out, using appropriate reagents, or several assays for the same analyte in one or more samples may be carried out simultaneously.

The main advantage of a guide means in the form of a channel in an enclosure is that this provides the simplest path along which the substrate may be directed through the housing means, so providing for the simplest and most easily automated analytical procedure. This arrangement is also well suited for use with transfer and wiping means in the form of absorbent pads which may be mounted linearly spaced along the channel. An enclosed channel can also provide a high degree of protection against the ingress of contaminants and isolates potentially harmful analyte on the substrate from the enviroment during analysis. Simplicity of construction and low cost hence disposability are other advantages. The enclosures may be made from two or more longitudinal enclosure portions to facilitate formation of the channel. These portions may be made from a thermoplastic material using injection moulding methods. The portions may be fitted together by snap-fit connectors, adhesive, ultrasonic welding, clips or other fastening means. A releasable or detachable fastening means has the advantage that the enclosure may be disassembled for internal decontamination, for changing the wiping and/or transfer means, or for insertion or removal of the solid substrate. Detent means adapted to cooperate with corresponding detent means on the solid substrate may be provided in the channel for accurate linear positioning of the surface portion adjacent liquid transfer means. Non-return means may also be provided in the channel, such as a ratchet device, to allow the substrate to move in one direction only thus ensuring the correct analytical procedure is followed.

According to a second aspect of the present invention there is provided a kit for detection and/or quantitative measurement of an analyte immobilised on an active surface portion of a solid substrate, which comprises;

an assay apparatus in accordance with the first aspect of this invention, and

a solid substrate, capable of being housed within the housing means of the assay apparatus and having an active surface portion for immobilising thereon analyte present in a sample solution.

The solid substrate, which is preferably of an elongated form such as a reactive stick or strip widely used in analytical chemistry and biochemistry, will usually be made of a plastic material or derivatised plastic material such as polyvinyl chloride (PVC) polystryrene, cellulose acetate, polycarbonate, polyacrylates or nylon etc. Analyte entrapment may be by physical or chemical interaction. Physical entrapment may be provided by a surface portion which has a porous structure thereby entrapping analyte by absorption and/or adsorption. However, a surface portion having a solid rather than porous structure is preferred to promote efficient wiping by the wiping means of the apparatus, in which case entrapment is preferably chemical and the material of the substrate in the region of the active surface portion is preferably of a type which can be readily activated chemically. The use of such materials in analytical chemistry or biochemistry will be well known to those skilled in the art. The substrate may be made from a solid, single material or it may be in the form of a laminate or two or more materials to give good handling and sensitising properties. Generally, where the substrate is in the form of a stick or strip, the active surface portion of the stick or strip may preferably be located in one or more specific defined areas of the stick or strip. On one or more other surface portions to act for example as an internal standard, for example containing a known quantity of an analyte or a substance that will react to a known extent with the first and/or second reagent to emit a signal. The use of such standards will be described later.

Conveniently one part of the substrate may be formed into a handle.

The properties and hence choice of the solid substrate material underlying the active surface portion thereof will depend largely upon the method used to detect the signal ultimately emitted from this surface portion after use in the present apparatus. If the surface portion emits an optical signal, either directly or under the influence of external stimulation, the material may be substantially transparent, translucent, opaque or reflective to the emitted signal and/or to incident radiation (eg uv light, x-rays or other photons, or electrons) which may be used to excite the surface portion into emitting a detectable light signal eg where the first reagent is a fluorescent material which binds to the analyte. Where the emitted light signal is from a luminescence reaction between the first and a second solubilised reagent, the material is preferably substantially transparent so that the signal can be detected through the substrate while second reagent is being transferred to the reactive surface portion; however in this case at least part of the rest of the substrate not underlying the reactive surface portion may be opaque to prevent ambient light being transmitted through the substrate from outside the holding means, so preventing outside interference with the detection of the luninescent reaction. A substantially transparent material is also required when the emitted signal consists of incident radiation which has been partly absorbed or scattered by the surface portion after treatment in the apparatus.

Where analyte immobilisation using a chemically, activiated surface portion is employed, the active surface portion of the solid substrate, which is preferably substantially planar to facilitate reagent transfer and surface wiping, will be chemically activated before use. If the kit is to be used in an enzyme immunoassay, the surface portion will preferably be activated by coating it with a layer, preferably a monolayer, of antibodies having specificity for the analyte. If the kit is to be used in a DNA assay the surface portion will preferably be activated by coating it with a layer of DNA complementary to the DNA analyte. If the kit is to be used for chemical analysis the surface portion will preferably be activated by coating it with a layer of a chemical reagent, for example a complexing agent.

Where the assay apparatus includes a guide means for guiding the solid substrate through the housing means, in order to provide for easy passage of the substrate through the housing means, the leading edge of the substrate preferably presents an inclined face to the transfer and wiping means. For example, where the substrate is in the form of a substantially flat stick or strip, the leading edge of the stick or strip may be rounded or chamfered on its face opposing the reagent transfer and wiping means. When the leading edge of the substrate is chamfered in this way, the wiping means may more easily wipe the leading edges and this has been found to be desirable.

The solid substrate may also include disengageable detent means which cooperate with corresponding detent means on the holding means to latch the substrate at the one or more predetermined positions to enable, for example, accuratley-positioned transfer of reagent and/or sample to take place. The detent means on the substrate may comprise one or more openings or recesses which cooperate with a resiliently biased plunger means of the housing means.

The substrate may be supplied in the kit fitted within the assay apparatus, or supplied separately in which case the user will insert the substrate into the apparatus. If the assay apparatus is designed to be reusable, the kit may contain one assay apparatus and several usable substrates adopted for use therein; if not, the number of substrates and apparatus in the kit will normally be the same. Each substrate or substrate housing means may incorporate some identification means such as the name of a patient or the test required.

As mentioned earlier, the liquid transfer means within the apparatus may contain lyophilised first reagent. Alternatively, the solublised first reagent may be applied to the apparatus externally, in which case the kit may also include solubilised first reagent supplied in a separate container. Other reagents, when required in the analytical procedure, may also be supplied in separate containers within the kit. When the reagent transfer means comprise absorbent pads containing an impregnated lyophilised reagent, the kit may also contain one or more of such pads, containing the same or different reagents, and adapted to be fitted into the apparatus of the first aspect of the invention as described above. Such pads may be supplied separately for use in the kit.

According to a third aspect of the present invention, there is provided a method of using the apparatus according to the first aspect which comprises detecting and/or quantitatively measuring an analyte immobilised on a surface of a solid substrate by the steps of;

(a) positioning the surface of the substrate at a first predetermined position within a substrate housing means,

(b) coating the surface of the substrate at the first position with an amount of a solubilised first reagent that binds to the analyte, the amount of first reagent being in excess to that required for binding to all the immobilised analyte,

(c) advancing the substrate through the housing means to displace the surface from the first to a second predetermined position,

(d) wiping excess unbound reagent from the surface of the substrate as the substrate is advanced to second position, by contacting the said surface with a substrate wiping means mounted within the housing means,

(e) coating the surface of the substrate at the second position with an amount of a second solubilised reagent, that reacts with the first reagent to produce a detectable signal, the amount of second reagent being sufficient to react with all the bound first reagent, and

(f) detecting the emitted signal.

The emitted signal may be light from a chemi- or bio-luminescent reaction between the first and second reagents.

The method may form part of an enzyme immunoassay, in which case the analyte will consist of an antigenic particle, especially a biological particle such as an antigenic protein or polypeptide, hapten, hormone and the like, and the first reagent will be an enzyme-labelled antibody. The second reagent may be a reagent that produces a chemi - or bio-luminescence reaction with the enzyme. Such enzyme-second reagent combinations will be well known to those skilled in art. The intensity of the light signal emitted by a luninescent reaction provides a measure of the amount of labelled antibody hence analyte bound to the substrate.

The method may alternately form part of a DNA assay, in which case the analyte will consist of a polynucleotide, and the first or second reagent may comprise labelled DNA complementary to the sequence of the analyte. The method may alternatively form part of a chemical analysis, in which case the analyte may be a metal ion or chemical radical, and the first and/or second reagents may be complexing agent(s). The method preferably also includes a method of immobilising analyte onto the surface of the substrate prior to step (a) comprising:

(i) positioning the surface of the substrate at a third predetermined position within the housing means,

(ii) coating the surface of the substrate at the third position with sample solution suspected of containing the analyte,

(iii) advancing the substrate through the housing means to displace the surface from the third to the first predetermined position, and

(iv) wiping unbound sample solution from the surface of the substrate as the substrate is advanced to the first position, by contacting the said surface with a substrate wiping means mounted within the housing means between the third and first predetermined positions.

Again, where the method forms part of an enzyme immunoassay, the substance contained within the surface of the substrate will be antibody, preferably deposited as a layer on the substrate, to the antigenic analyte.

For convenience of use and ease of automation, the path of the substrate from the first to the second and from the third to the first positions, preferably each describes a substantially straight line. More preferably, the path of the substrate from the third to the first to the second position describes a single straight line. The wiping means are preferably absorbent pads. Sample and/or reagent solutions are preferably transferred to the substrate surface through absorbent pads. Step (f) preferably consists of detecting the emitted signal through the solid substrate while second reagent is being transferred to it at the second predetermined position, in which case the substrate will be substantially transparent to the emitted signal.

It will be appreciated by those skilled in the art that the emitted signal from the solid substrate may comprise an optical signal, for example a change in colour, which can be observed and assessed by the naked eye. In this instance the present assay apparatus requires no ancillary signal detection and measurement equipment, in which case the assay apparatus may comprise a complete analytical device. It will also be appreciated that emitted signal detection and measurement may be effected after withdrawing the solid substrate from the substrate housing means. However, where the present apparatus includes a means for directing a particulate emitted signal out of the substrate housing means so that signal detection and measurement may be effected without removing the solid substrate from the substrate housing means, then there is preferably additionally provided an instrument for detecting and measuring an emitted particulate signal which is specifically adapted to be releasably combined and used with the present apparatus.

According to a fourth aspect of the present invention, therefore, there is provided an analytical device comprising the assay apparatus according to the first aspect having means for directing a particulate emitted signal from the substrate housing means, and a signal measuring device comprising particulate signal detection means, detector housing means housing the detection means and adapted to receive the substrate housing means of the assay apparatus, detected signal amplification means, amplified signal processing means, data storage and/or display means, and location means for locating the substrate housing means in the detector housing means at a signal receiving position such that the detection means is in signal receiving alignment with the means for directing the emitted particulate signal.

The detection means may be adapted to detect a particulate signal, especially photons, consisting of fluorescence, luminescence, transmitted, reflected, or scattered light, beta backscatter, x-ray fluorescence, or isotopic radiation emitted from the surface portion of the substrate when the analytical device is being used. Luminescence may be detected using photomultipliers used in either photo current or photon counting mode. A more convenient detection means for detecting luminescence is to use silicon photodiodes, again in either photo current or photon counting mode. The use of such means is well known to those skilled in the art. However, one photodiode may not be ideally suited to both modes of operation, hence separate photo current and photon counting detectors may be provided.

Where optical fluorescence, or reflected, transmitted or scattered light is being detected, the signal measuring device will preferably include a source of particulate stimulant, especially photons, for directing at the solid substrate. This source will be aligned with the means for directing particulate stimulant onto the solid substrate by the location means. Typical sources of photons which may be used are tungsten lamps, continuous or pulsed xenon lamps, and lasers. The detection means may be similar to that described above for detecting luminescence and appropriate additional optical components such as filters, lenses and mirrors may also be required. Such optical arrangements are well known to those skilled in the art.

The detector housing means preferably includes screening means for protecting the detection means from external sources of particulate signals, especially ambient light photons. For example, the detection means is preferably housed within a housing means comprising an opaque enclosure. Screening of the detection means from ambient light is particularly relevant to luminescence, light absorbance, fluorescence and light scattering methods of detection. Ambient light can cause photomultipliers and silicon photodiodes to drift and can destroy active photomultipliers. For this reason, the signal measuring device may additionally include shutter means which exposes the detection means to the means for directing the particulate emitted signal when the substrate housing means is located at the signal receiving location within the detector housing means.

A detector housing means which is preferred when the emitted signal consists of optical photons, especially photons from a luminescence reaction, comprises a detector housing member having an anlaytical chamber therein adapted to contain the signal detection means, a recess in the detector housing member which communicates with the analytical chamber, the recess having guide means therein for receiving the substrate housing means and guiding it towards the analytical chamber. The signal detection means is preferably located in very close proximity to the recess hence position of the solid substrate to maximize the magnitude of the received signal. The detector housing member is preferably at least partly opaque to optical photons in order to screen the analytical chamber. The substrate housing means is preferably also opaque to optical photons.

In one embodiment of the present analytical device, the shape of the recess in the preferred detector housing means preferably corresponds closely to that of the substrate housing means so that a close fit between the two is obtained when the latter is fully inserted within the former. Shutter means are preferably provided mounted within the detector housing means between the detection means and recess. Hence, initial insertion of the substrate housing means into the recess blocks off most ambient light to the analytical chamber. Further insertion causes the substrate housing means to coact with the shutter means which in the absence of the substrate housing means closes the communication between the recess and the analytical chamber. Still further insertion causes the shutter means to open, hence exposing the means for directing the emitted signal to the detection means within the analytical chamber. The insertion preferably also causes the shape of the substrate housing means to coact with the corresponding shape of the recess to form a light seal. Removal of the substrate housing means reverses the action of the shutter means which preferably includes resilient biasing means such as a spring to close the shutter means in the absence of the substrate housing means.

In a further embodiment of the signal measuring device, the assay apparatus is detachably mounted within a carrier means which is slideable within the recess in the preferred detector housing means. The carrier means thereby acts as a cartridge carrier for the assay apparatus to facilitate its insertion into and withdrawal from the detector housing means. The carrier means is preferably opaque and a close fit within the recess in the detector housing means so that it may additionally act as the shutter means, provided that the carrier means is designed so that loading and unloading of the assay apparatus may be effected while the carrier means is still at least partly inserted within the recess. The provision of a separate shutter means in the present measuring device is thereby rendered unnecessary.

It will be understood that, in use, treatment of a solid substrate in the assay apparatus may be effected either after or, preferably, before insertion of the substrate housing means into the detector housing means. It is important that the means for directing the emitted signal is accurately aligned with the detection means and accordingly a location means should be provided.This may consist of a detent means in the substrate housing means that engages a resiliently-biased member, for example a spring-loaded ball, in the recess of the detector housing means when the correct position has been attained. Alternatively, the detent means may be in the recess and the biased member on the substrate housing means. This resiliently-biased location means is preferably positioned so that it also urges the substrate housing means towards the detection means to improve light sealing. As a yet further alternative, where the assay apparatus is mounted within a carrier means, the location means may consist of an accurately positioned mounting on the carrier means to receive and accurately position the assay apparatus in the carrier means, and an insertion stop means on the detector housing means which prevents further insertion of the carrier means into the detector housing after correct alignment with the detection means has been attained. A further means is preferably also provided for detecting the correct position of the substrate housing means within the detector housing means and signalling the signal measuring device to begin to take readings. This further means may consist of a switch actuated by the location means, by the substrate housing means or (when provided) by the carrier means.

In the present analytical device, it may sometimes be desirable to check the performance of the device either by using a stable light emitting means which provides a standard light source, or by comparing the readings from the device produced from a known and from an unknown light signal. Chemical methods of producing stable light emissions (ie by luminescence) are not sufficiently constant, particularly on a long term basis, because the characteristics of different batches of biological chemicals can vary. Physical light sources are more stable and accordingly the signal measuring device may incorporate such a light standard which can be directed at the detection means. This physical light source may consist of a conventional light source, such as a filament lamp or light emitting diode, which is powered from a battery or regulated power supply. Alternatively the standard light source may consist of a scintillant which is activated by a radioactive source, such as carbon 14 or tritium gas, to produce a substantially constant light emission. Where a standard light source is provided in the present measuring device, the device preferably includes a means for selectively exposing the detection means to total darkness, to the standard light source and to the light signal being emitted from the luminescence reaction on the solid substrate. The standard light source is preferably incorporated into the shutter means so that by appropriate operation of the shutter means the detection means is exposed to these three light conditions at different shutter positions.

The present analytical device is ideally suited for extra-laboratory testing such as in a doctor's surgery, in a hospital ward or for vetinary use on a farm. However, testing outside the laboratory usually makes normal quality control methods impracticable. The use of a standard light source, as described above, may be used to over-come this problem to some extent. However, a better solution may be provided by incorporating a chemically-produced light standard in the present analytical device which is generated simultaneously with the luminescence reaction on the substrate surface on which analyte from the sample is immobilised.

For example, two assay apparatus, each preferably having a linear arrangement of reagent transfer and wiping pads disposed therein, may be combined into a single module but share a common solid substrate. One area of the substrate may be coated with a sample solution containing an unknown amount of analyte while a second area of the substrate may be precoated with a known amount of analyte which can then provide an internal standard.

The solid substrate may then be advanced through both apparatus simultaneously to effect treatment of the one area by one of the apparatus and the second area by the other apparatus. Hence, a light signal may be produced from each of the two areas, the magnitude of the two signals relating to the amount of analyte in the sample and in the standard respectively. The detection means is preferably arranged to detect both of these two signals, and a microprocessor is preferably arranged to receive, analyse and respond to the resulting signals emitted from the detection means. If the magnitude of output from the detection means resulting from the detection of the emitted signal from the standard is outside predetermined limits, the microprocessor is preferably arranged to suppress the result and generate an error signal which the device preferably subsequently displays. Hence, the use of built-in chemical standards greatly reduces the possibility of error caused by the analytical device, the operator of the device or the environment.

Error detection may be further improved by also monitoring the signal from a physical light source, as described above. This may further enhance the reliability of the analytical device in the sense that the only circumstances under which an error would not be detected would be in the unlikely event of the device, the chemical standard and the physical standard all failing together.

Methods and apparatus for automating the use of the apparatus and the kit in the method of the invention will be apparent to those skilled in the art. For example a device may be cosntructed using known timing means etc for directing the substrate into the first and second predetermined positions, retaining it there for an appropriate period of time and then detecting the emitted signal.

The present invention will be described in greater detail by way of example only with reference to the accompanying drawings, in which

Figure 1 is a plan view of a assay apparatus of the invention, showing lines $AA^1$, $BB^1$ and $CC^1$ and having a substrate strip fully advanced within it,

Figure 2 is longitudinal sectional view of the apparatus of Figure 1 taken along $AA^1$

Figure 3 is a longitudinal sectional view of the apparatus of Figure 1 taken along $BB^1$

Figure 4 is a side elevation of the apparatus of Figure 1,

Figure 5 is a cross sectional view of the apparatus of Figure 1 taken along $CC^1$,

Figure 6 is a sectional view of an assay apparatus of the present invention employing a solid substrate in the form of an endless strip,

Figure 7 is a perspective view of an assay apparatus consisting of a mutliple holder in which a strip is inserted and removed from each pad in turn,

Figure 8 is a cross sectional view of the holders of Figure 7 in an open position,

Figure 9 is an exploded diagrammatic view of assay apparatus consisting of holders containing wiping pads, in which conventional vessels are used instead of pads to coat the strip with liquid,

Figure 10 is a plan view of an analytical device incorporating the apparatus illustrated in Figures 1 to 5 positioned within it, showing line $DD^1$,

Figure 11 is a longitudinal sectional view of the device of Figure 10 taken along $DD^1$, and showing

further line FF[1],

Figure 12 is a longidutinal sectional view of the device of Figures 10 and 11 taken along FF[1] and showing further line GG[1]

Figure 13 is a cross-sectional view of the device of Figures 10-12 taken along GG[1],

Figure 14 is a plan view of an assay apparatus suitable for use with a transparent substrate strip, showing lines II[1] and JJ[1],

Figure 15 is a longitudinal section view of the apparatus of Figure 14 along line II[1],

Figure 16 is a cross-sectional view of the apparatus of Figure 14 along line JJ[1],

Figure 17 is a horizontal section taken along line LL[1] of Figure 18 of an analytical device incorporating the apparatus illustrated in Figures 14 to 16 and showing line KK[1],

Figure 18 is a vertical section of the device illustrated in Figure 17 taken along line KK[1] and showing line LL[1].

Figure 19 is a plan view of any assay apparatus suitable for use with a transparent substrate strip, showing lines MM[1]

Figure 20 is a longitudinal sectional view of the apparatus of Figure 19 along line MM[1], and showing lines NN[1] and OO[1],

Figures 21 and 22 are a cross sectional view of the apparatus of figures 19 and 20, about lines NN[1] and OO[1] respectively

Figure 23 is an exploded perspective view of the apparatus of Figures 19-22

Figures 24 and 25 are standard curves from assays performed with the apparatus of Figures 19-23 in which the quantity of mouse IgG and human chorionic gonadotrophin (HCG) respectively in a sample are plotted against the resulting light output.

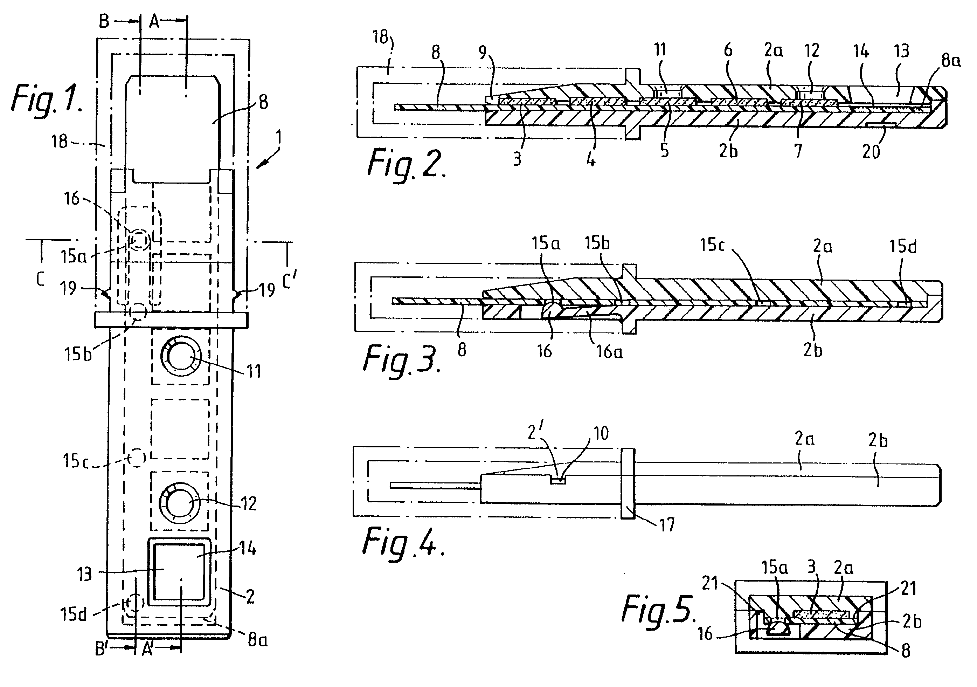

Referring first to Figures 1 to 5, these show an assay apparatus 1 in use with a rigid reactive strip 8 fully inserted within it. The strip 8 is made of an opaque plastic material such as PVC onto one end of which is immobilised an analyte-bindable layer of, for example, proteins to form a sensitised surface 14 adjacent forward edge 8a. The strip 8 is slideable in a holder 2 which is made of an opaque, inert thermoplastic material. The holder 2 houses a sample addition pad 3, first wiping pad 4, first reagent addition pad 5, second wiping pad 6, and second reagent addition pad 7. Each of these pads is made from a resilient, opaque, absorbent, inert material such as cellulose-based filter paper. Pads 4-7 are located so that the material of the pads is slightly compressed by the passage of the coating surface of strip 8 as it is inserted into the holder 2. The forward edge 8a of strip 8 is chamfered to allow easy movement of the strip 8 past the pads 4 to 7. The length and width of each pad 4-7 extends just beyond that of the area 14 of strip 8 to ensure total coverage. The edge of thin, porous sample pad 3 is open to the outside of the holder 2 at 9 and channel 10 acts as a vent (Figure 4).

Pad 3 is located in the holder 2 so that its surface just touches or is a small distance from the adjacent surface of strip 8. This ensures that when the sample pad 3 is saturated with sample liquid applied at the edge 9 of the pad extending just outside holder 2, surface tension effects cause even coating of area 14 with sample liquid when strip 8 is pushed into holder 2. The edge 9 of pad 3 may incorporate a filter means to separate out unwanted material from the sample to prevent it reaching area 14 of strip 8. For example, where the sample is whole blood it may permit the passage of serum or plasma only. Alternatively, a separate filter means (not shown) may be provided in the path of the applied sample before it reaches the sample pad 3.

Reagent addition ports 11 and 12 connect pads 5 and 7 respectively to the outside of holder 2. These allow the pads to be saturated with liquid reagent chemicals, or lyophilised reagents in the pads to be activated with added liquid. Aperture 13 connects the chemically active area 14 of strip 8 to the outside of holder 2 allowing access by the detection means of a measuring instrument (not shown). The walls of aperture 13 may be made reflective to increase light collection efficiency from the area 14 of the strip. The strip 8 may be made reflective at area 14, for example by including a reflective metallic coating or metallic insert on the strip 8 at area 14 underlying the analyte-bindable layer.

Strip 8 is provided with holes 15a,b,c and d. These holes engage a cooperating detent 16 on a resilient finger 16a, to provide a positive means of locating the strip within holder 2 at a series of semi-fixed predetermined positions as it is advanced into the holder, (Figs 3 and 5). Finger 16 is moulded integrally with the holder 2, the material of which has some degree of resilience.

The holder 2 is provided with a flange 17 which fits against a cooperating portion on a signal measuring device (not shown) to form a seal against the ingress of light. A cap 18 (shown in dotted outline) is provided to prevent light leakage into the holder 2 and to form a biological safety cover over sample application area 9. The cap, which is made from an opaque, resilient thermoplastic material, is located against flange 17 and is prevented from inadvertent removal by detents 19. Recess 20 is used to locate the holder accurately within the measuring device, where it engages a cooperating catch (not shown). The holder 2 is preferably made in two halves (2a, 2b) so that the internal structure can be formed. Locations 21 serve to align the two halves which may be joined by adhesive, ultrasonic welding or integrally formed clips (not shown).

In use, the leading edge 8a of strip 8 is advanced into holder 2 past pads 3, 4, 5, 6 and 7 in turn until it reaches the position shown in Figures 1 to 5 with area 14 directly under aperture 13. Strip 8 is first inserted into holder 2 until area 14 is situated directly beneath pad 3. Sample liquid suspected of containing a particular analyte is then applied to edge 9 of pad 3. The sample can be applied using a pipette or, in the case of assays using whole blood, from a thumb prick using typically 10 μl of blood. As pad 3 becomes saturated with liquid, liquid is transferred evenly to area 14 of strip 8 by surface tension effects. Ideally, area 14 is slightly larger than the "contact" surface area of pad 3 to ensure that all the analyte within the sample liquid is exposed to area 14. Area 14 remains in contact with pad 3 for a sufficient incubation period to permit analyte present in the sample to react with and "bond" onto area 14. In the case of an enzyme immunoassay, antigen in a liquid sample of blood, serum or other body fluid or tissue is attracted to and "bonded" to a coating of an appropriate antibody on area 14, for a period of typically 15 minutes.

When the incubation time is finished, strip 8 is advanced slowly through the holder 2 so that the area 14 is wiped by the first wiping pad 4 to remove excess sample liquid, until area 14 is directly beneath pad 5. It will be appreciated that movement of strip 8 may be undertaken manually or automatically. The wiping pad 4 may be used in a dry state but for some assays it may be necessary to moisten it with a suitable solvent applied, for example, through a hole (not shown) connecting the pad 4 to the outside of the holder 2 from an appropriate delivery means (such as a syringe).

Once area 14 is beneath pad 5, excess solubilised first reagent contained within pad 5 and capable of reacting with immobilised analyte is transferred to area 14. The pad 5 may be saturated with this solubilised reagent either before or after it comes into contact with area 14. Pad 5 may be saturated with reagent using a syringe to deliver reagent through hole 11 or, alternatively, using a syringe to deliver a solubilising solvent (eg distilled water) through hole 11 onto pad 5 which has previously been impregnated with lyophilised reagent. Since it is known that certain lyophilised reagents are unstable, to overcome this problem the pad 5 may comprise a laminate of two or more pads containing different lyophilised reagents. In this case reconstitution also causes mixing of these different reagents by surface tension and dispersion effects. In a typical immunoassay, the reagent consists of an enzyme-labelled antibody to the antigenic analyte and area 14 and pad 5 remain in contact for an incubation period of typically 15 minutes to allow the antibody (and hence enzyme label) in the reagent to be attracted and "bonded" to the analyte immobilised on area 14.

Then this further incubation time has finished, the strip 8 is advanced further into holder 2 so that area 14 is then wiped by second wiping pad 6 to remove excess unreacted solubilised reagent. At this point, the amount of reagent that has reacted with the analyte may be determined by advancing strip 8 until area 14 is under aperture 13, in which case pad 7 will contain no active reagents. For example, the reagent transferred through pad 5 may be a fluorescent-labelled antibody which can be detected without the addition of further reagent. Suitable fluorescent labels for antibodies will be well known to those skilled in the art, and include fluorescein derivatives and rhodamine derivatives. Alternatively, a second solubilised reagent may be transferred to area 14 from pad 7. In this case, strip 1 is further advanced into holder 2 until area 14 is directly beneath pad 7. Pad 7 is then saturated with excess second reagent using, for example, a syringe to deliver a solubilising solvent (eg distilled water) through hole 12 onto pad 7 which has previously been impregnated with dry lyophilised second reagent. This second reagent may be employed to react with the bound first reagent to produce a signal-emitting reaction or reaction product. In the case of an enzyme immunoassay, the second reagent may react with the enzyme "bonded" to area 14 to produce, for example, an amount of light from a chemi- or bioluminescence reaction which is dependent on the amount of analyte in the sample.

The strip 8 is then advanced until area 14 is directly beneath aperture 13, which then allows examination of the labelled area 14 to be undertaken.

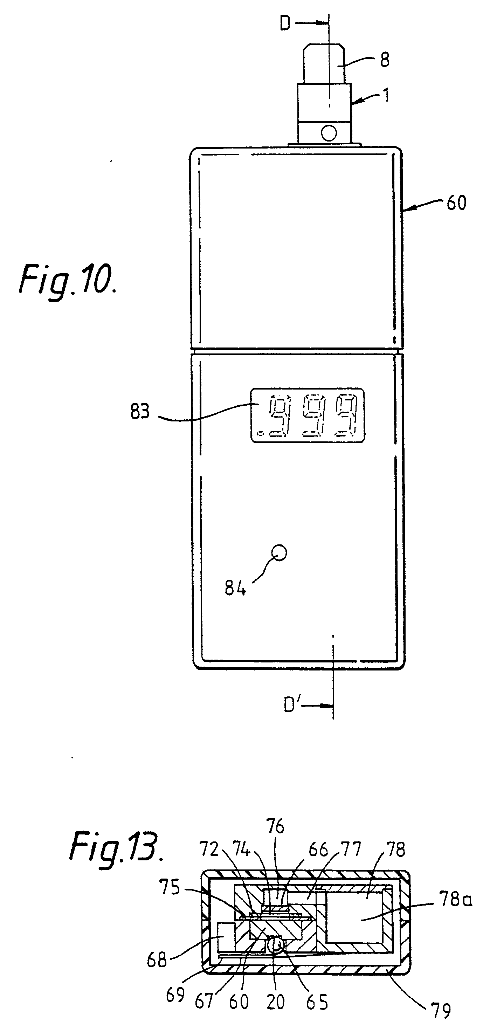

A description of one specfic use of the first embodiment of the present assay apparatus will follow the description of the measuring instrument illustrated in Figures 10 to 13.

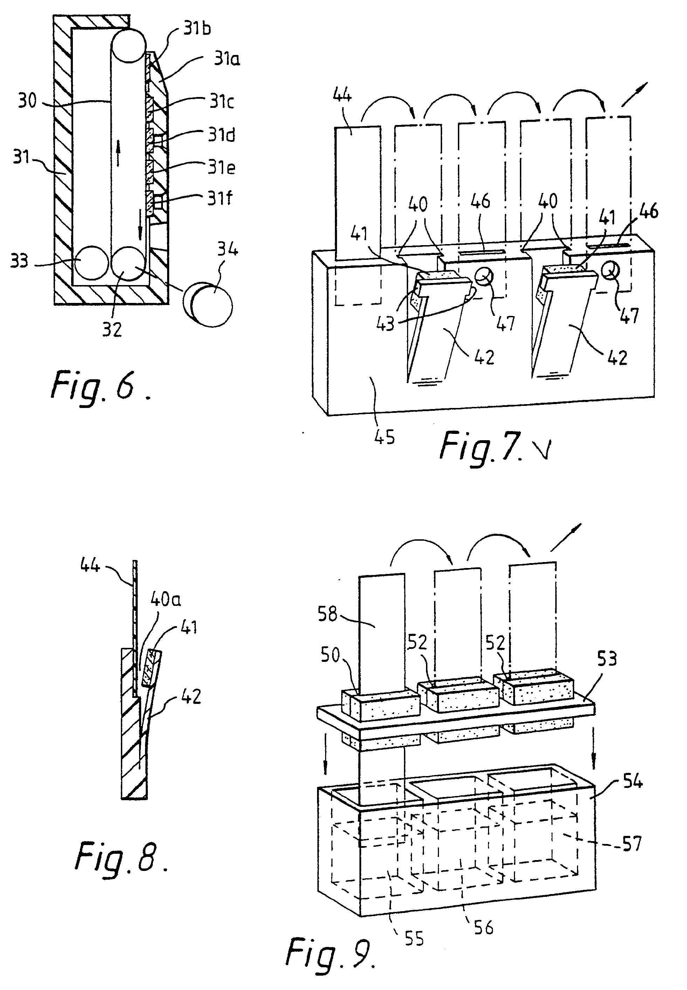

Figure 6 illustrates a second embodiment of the invention similar to the first, in which a flexible strip 30 is housed within a holder 31. A portion 31a of the holder 31 is similar in construction to the top half of 2a of the holder 2 illustrated in Figures 1 to 5. The strip 30 is driven past the various pads 31b, 31c, 31d, 31e & 31f by a pinch wheel 32 and idle wheel 33. The pinch wheel 32 is connected to a knob 34 projecting outside the holder which is turned manually. It will be understood that this arrangement could form the basis of an automatic machine in which the strip 31 is fed automatically past the various pads 31b, 31c, 31d, 31e & 31f In this case, the device would preferably include a means for rejuvenating the sensitised area (not shown) of the strip 30 after sample detection has been carried out.

Referring now to Figures 7 and 8, these show a third embodiment of the assay apparatus of the invention in which a strip 44 is inserted into and withdrawn from the sample and each wiping and reagent pad in turn in the direction of the arrows shown. In this embodiment, the wiping pads must be clear of the strip during insertion and close onto the strip prior to withdrawal. Channels 40 guide a strip 44 into wiping recesses 40a set within a multiple holder 45. A wiping pad 43 is fixed to a spring loaded lever 42 which, in its relaxed position, holds the pad 41 away from the strip 44. The lever 42 is shown in Figures 7 and 8 as forming an integral part of the holder 45 which is preferably moulded from an opaque, resilient, thermoplastic material. To wipe the strip 44, it is urged down along channels 40 into recess 40a, lever 42 is manually pressed towards the holder 45 and hence the pad 41 pressed into contact with the strip. The strip 44 is then withdrawn and lever released. Flanges 43 on the lever 42 coact with the holder 45 to form a stop to limit the compression of the pad 41. The strip 44 is then inserted into a reagent addition recess 46 in the holder 45, having reagent addition port 47 and reagent pad (not shown), before being moved on to the next recess 40a.

Referring next to Figure 9, this shows a fourth embodiment of the assay apparatus in which the specimen and reagent addition means comprise conventional vessels. Wiping pads 50, 51 and 52 are incorporated into lid 53 which, in use, fits onto vessel 54. Vessel 54 has three compartments 55, 56 and 57 containing a sample and two reagents respectively. In this embodiment the wiping pads 50, 51, 52 wipe both sides of a strip 58. In use the strip 58 is inserted through each pad and into each compartment in turn.

Referring next to Figures 10 to 13, these show a first luminescence measuring device 60 fitted with a removable assay apparatus 1 which is identical to that illustrated in Figures 1 to 5. A chamber 61 formed in block 62 is made light-tight by the flange 17 of the apparatus 1 coacting with a recess 63 at the entrance to the chamber. A resilient light-tight gasket 64 between flange 17 and recess 63 provides additional protection against the ingress of light. The apparatus 1 is located accurately within block 62 by a spring loaded ball 65 acting against recess 20. Furthermore, ball 65 presses the apparatus 1 against the opposite wall of chamber 61 to further effect ambient light sealing in the region of light transferance between the analytical device and a light detector 66. Leaf spring 67 biases ball 65 towards apparatus 1 and extends to a microswitch 68 so that when the ball 65 engages the recess 20 (ie when the apparatus 1 is in its correct position in the instrument), the moving end 69 of the spring 67 actuates the microswitch. This signals a microprocessor 70 to take the necessary action. The leading edge of the apparatus 1 coacts with a projection 71 on a shutter 72 when the device is correctly positioned. In the absence of apparatus 1 the shutter 72 is biased by torsion spring 73 to close aperture 74 in aperture plate 75 connecting chamber 61 to detector 66. The shutter 72, aperture plate 75 and detector holder 76 coact to form a labyrinth to prevent ambient light entering detector 66.

The detector 66 is preferably a silicon photodiode the output of which is connected via transverse aperture 77 to a highly sensitive electronic amplifier 78 housed in chamber 78a. It is important that the detector 66 and amplifier 78 are properly screened from outside radiation and the structure containing chambers 61 and 78a should preferably be made of aluminium or other appropriate screening material.

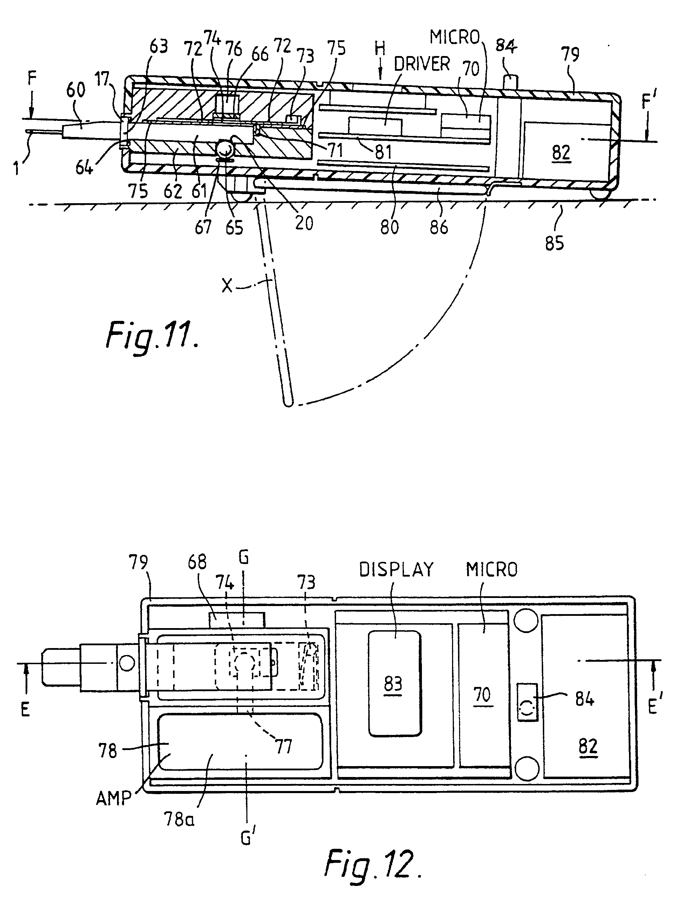

The chambers 61 and 78a are housed in case 79 which also houses electronics circuit boards 80 and 81, battery housing 82, display 83 and start button 84. The case 79 may be hand held, used flat on a bench 85 in the position shown in Figure 11, or rested on a retractable stand 86 when in its open position X illustrated by dotted lines in Figure 11.

The use of the apparatus 1 within the measuring device 60 illustrated in Figures 10-13 will now be described using a typical luminescent enzyme immunoassay as an example. Antibodies specific to a virus, such as rubella, are first immobilised onto strip 8 to form the sensitised area 14. It is only necessary to coat a short length at one end of the strip 8. The length coated should be a little longer than the length of a pad 3-7. This coating may conveniently be done by the manufacturer of the apparatus 1. The strip 8 is then inserted into the holder 2 so that the sensitized, chemically active area 14 is beneath the sample application pad 3. Approximately 10 μL of a whole blood sample from a thumb prick which contains the analyte to be detected is placed onto the apparatus 1 at position 9. Surface tension causes the sample to be drawn into pad 3 and coat the sensitized area 14 of the strip 8. The strip 8 remains in this position for typically 15 minutes to allow antigen in the blood sample be attracted to and "bond" to the antibody of the strip. The strip 8 is then pushed into the holder 2 passed wiping pad 4 which removes excess sample from area 14, until the sensitive area 14 is directly beneath and in contact with pad 5. Typically, 80 μL of enzyme-labelled antibody is injected through hole 11 using a syringe to saturate pad 5. The amount of antibody injected is in excess of that required by the chemical reaction on the surface of area 14, and hence the exact quantity injected is not critical. The strip 8 remains in this position for typically 15 minutes to allow antibody and hence label to be attracted and "bonded" to the antigen of the analyte. The strip 8 is then pushed further into the holder 2 passed wiping pad 6 which removes excess liquid from the area 14, until the sensitive area is directly beneath and in contact with pad 7. Typically, 80 μL of reagent is injected through hole 12 to saturate pad 7. This reagent, which is again present in excess to that required, reacts with the enzyme label immobilised on area 14 to produce an amount of light which is related to the amount of rubella in the sample. The strip 8 is then pushed further into the holder 2 so that the now light emitting area 14 is opposite window 13. Holes 15a, 15b, 15c and 15d in the strip 8 engaging the cooperating detent 16 of the holder 2 help to ensure the area 14 is accurately positioned beneath each of the pads 3, 5, and 7 and the window 13.

When currently-available enhanced luminescent reagents are used, typically the light is substantially stable for up to 30 minutes. This gives adequate time to transfer the apparatus 1 into the measuring device 60 illustrated in Figures 10 to 13. When using reagent systems which produce a pulse of light, it is more practical for the light producing reaction to be initiated inside the measuring device 60.

Before the light is measured by the measuring device 60 certain preliminary procedures need to be initiated. If the device 60 has not been used for typically 30 seconds then it may be of a type that automatically switches to a "sleep" mode to conserve power. Switch 84 must then be depressed to "power-up" the instrument and typically 10 seconds are needed for the electronics to restabilize. Before introducing or initiating chemically produced light inside the instrument, a measurement, corresponding to total darkness on detector 66 may be stored by the device 60 the so called "dark current" measurement. This can be subtracted from a subsequent light measurement taken from the glowing area 14 on the strip 8, and hence correct for electronic drift due to temperature effects etc. The dark current measurement may be initiated automatically after completion of the powering-up time or it may be performed manually. Once the light-emitting reaction on the strip 8 has been initiated by the procedures outlined above, the apparatus 1 is then pushed into the suitably-primed measuring device 60. Initial insertion blocks the path of ambient light to the analytical chamber 61. Further insertion moves shutter 72, exposing the detector 66 to light from the area 14 of the strip 8. Complete insertion light-seals the apparatus 1 within the measuring device 60 and actuates switch 68 as the flange 17 abuts the gasket 64. Actuation of switch 68 signals the microprocessor 70 to store a light measurement from the detector 66 via the amplifier 78 and other electronics.

To improve the precision the dark and light readings are not merely single measurements but comprise either the mean of many measurements(typically 25)or, preferably, the integration of continuous measurements over a period of time, typically 10 seconds. Such a measuring system is also effective for purely chemical methods of analysis which produce pulses of light lasting typically 0.5 seconds.

In a typical luminescent enzyme immunoassay it is normal to relate the amount of analyte in a sample to a standard analyte of known value. This may be done manually in which case the instrument merely displays relative units of light (photons) received by detector 66, for example it may display "light" seconds. Alternatively, the device 60 may store calibration factors and hence display the amount of analyte in the sample in the appropriate chemical units..

Withdrawal of the apparatus 1 from the device 60 reverses the action of shutter 72 and, if the device 60 is not used again within typically 30 seconds, automatically reverts to the "sleep" mode.

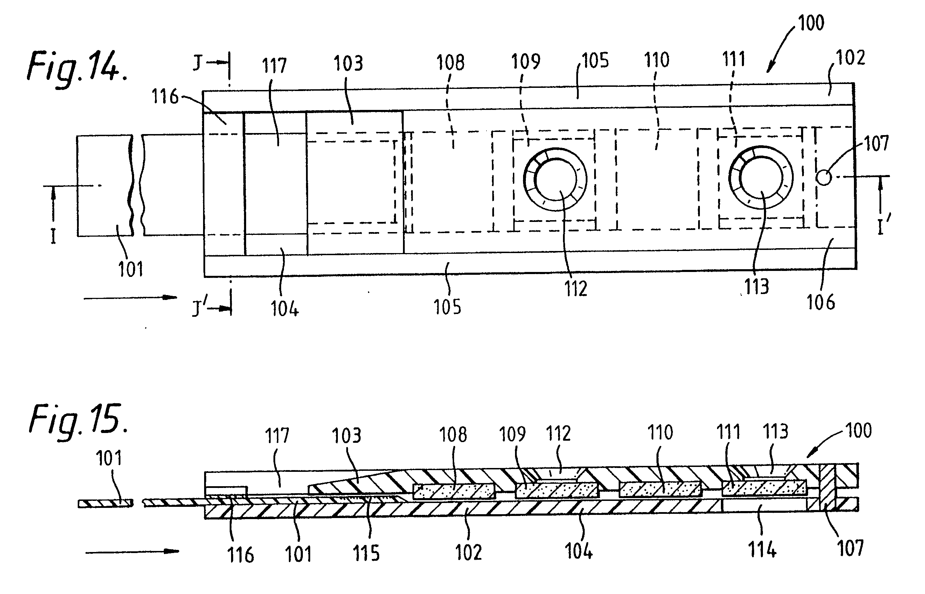

Referring next to Figures 14 to 16, these show a fifth embodiment of the present assay apparatus which has many features in common with the first embodiment illustrated in Figures 1 to 5, and comprises an assay apparatus 100 shown in use with a transparent rigid reactive strip 101 partly inserted within it. Apparatus 100 consists of a holder provided in two longitudinal holder portions 102 and 103. Lower holder portion 102 is provided with a longitudinally recessed base 104 and upwardly extending sidewalls 105 which together form a channel 106 into which upper holder portion 103 fits. These sidewalls help to prevent sideways loss of analyte and reagent (in addition to surface tension effects mentioned earlier) and so reduce the risk of contamination. A peg 107 passing through both portions 102 and 103 at one end longitudinally locates portion 103 in channel 106. Upper portion 103 is provided with a first wiping pad 108, first reagent addition pad 109, second wiping pad 110, second reagent addition pad 111 and reagent addition ports 112 and 113 similar to holder portion 2a of the labelling apparatus illustrated in Figures 1 to 5. Lower portion 102 is provided with an aperture 114 opposite second reagent adition pad 111. Strip 101 is provided with a chemically active area 115. Liquid transfer onto, and wiping of the area is effected by urging the strip 101 along the recess in base 104 between holder portions 102 and 103 in the direction indicated in Figures 14 and 15, in a manner which is similar to that described above for the assay apparatus illustrated in Figures 1 to 5. A bowed flat spring 116 grippingly engages the sidewalls 105 of lower holder portion 102 to urge the strip 101 against the base 104. The open area 117 between the spring 116 and upper portion 103 provides a sample addition polt through which sample solution may be transferred directly onto strip 101 at the appropriate moment. The position of aperture 114 allows any signal emitted from area 115 through strip 101 to be detected outside the assay apparatus 100 while area 115 is maintained in continuous contact with second reagent addition pad 111. This is a particular advantage when detecting luminescent reactions.

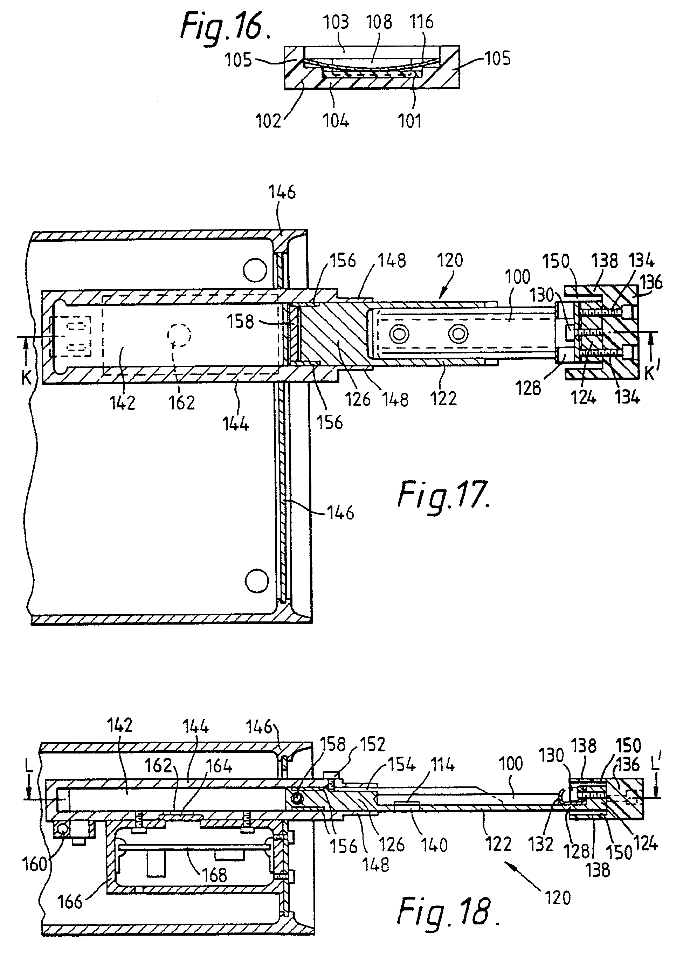

Referring now to Figures 17 and 18, these show a second luminescence measuring device 120, similar to the first measuring device 60 illustrated in Figures 10 to 13, fitted with the assay apparatus 100. The apparatus 100, shown in Figures 17 and 18 with the strip 101 fully inserted within it such that area 115 is in contact with second reagent addition pad 111, is mounted within an opaque slideable cartridge 122 having a tailpiece 124 and a headpiece 126. A curved spring 128 affixed to tailpiece 124 of the cartridge 122 by bolt 130 urges against the apparatus adjacent the flat spring 116 to hold the apparatus firmly in position within the cartridge between the tailpiece 124 and headpiece 126. Curved face 132 of spring 128 facilitates snap-fit retention of apparatus 100 within the cartridge 122. The outward face of tailpiece 124 is affixed by bolts 134 to the inside of an opaque cap 136 having sidewalls 138. An aperture 140 is provided in the cartridge 122 which is positioned so as to align with aperture 114 on lower holder portion 102 of apparatus 100 when the apparatus 100 is correctly mounted in the cartridge with spring 128 adjacent spring 116, thereby allowing any light generated by luminescence on area 115 of strip 101 to pass through the cartridge.

The cartridge 122 is slideably mounted within an elongated recess 142 within a housing member 144 whose open end protrudes from an instrument casing 146. The cartridge 122 is a close fit within recess 142 to help prevent the ingress of ambient light into the recess. The open end of the housing member 144 is provided wihth an outwardly-extending rectangular collar 148 which engages a correspondingly shaped recess 150 formed between the tail-piece 124 of cartridge 122 and sidewalls 138 of cap 136 when the cartridge is fully inserted within recess 142, thereby preventing ambient light reaching apparatus 100. A screw 152 extending through the housing member 144 close to its open end prevents inadvertent complete removal of the cartridge 122 from the recess 142 by abutting one end of a short groove 154 in the headpiece 126. The screw 152, together with a light-tight felt collar 156 about headpiece 126 additionally help to ensure that the recess 142 is always sealed against exposure to ambient light whatever the position of cartridge 122. The headpiece 126 also houses a permenant magnet 158 which activates a reed switch 160 at the closed end of the housing member 144 when the cartridge 122 is almost fully inserted within the recess 142. When activated, the switch 160 signals the device 120 to take readings. The cartridge 122 and housing member 144 are blackened to assist light-tightness and prevent light scattering.

The housing member 144 is provided with an aperture 162 in which a light detector 164, for example a silicon photodiode, is located. Aperture 162 aligns with aperture 140 and hence aperture 114 when the catridge 122 is fully inserted within the recess 142, thereby exposing the detector 164 to any light generated on area 115 of strip 101. The detector 164 is situated as close as possible to area 115 in order to maximise the sensitivity of the device 120, because the magnitude of the light signal detected is approximately inversely proportional to the square of the distance between the luminescent reaction on area 115 and the detector 164. Box 166 houses a high gain amplifier 168 which amplifies the signal received from the detector 164. Instrument casing 146, which encloses housing member 144 and box 166 and is designed to screen the interior of device 120 from external radiation, also houses other electronic and display components (not shown) as have been previously described for the first measuring device 60.

In use, strip 101 is first fully inserted into apparatus 100 so that light generated from a luminescence reaction on area 115 is emitted through aperture 114. The apparatus 100 is then snap-fitted into the cartridge 122. The electronic circuit of the device 120 is then activated by a button (not shown), and a "dark current" reading made, that is to say a reading taken with the cartridge in its position shown in Figures 17 and 18. Once this reading has been taken, the cartridge 122 is then pushed into recess 142 until fully inserted. Full insertion of cartridge 122 causes magnet 158 to activate reed switch 160 which in turn signals the device 120 to read the light emitted from the luminescence reaction on area 115. The dark current reading is then subtracted from the second reading and the result is a reading from which the amount of analyte present in the sample can be decuded

The advantage of the cartridge 122 in this second measuring device 120 may be seen from the above description of the construction and use of the device. Firstly, it obviates the need for a separate mechanical shutter to protect the detector. Secondly, a well engineered, relatively massive cartridge provides a more robust device construction and more efficient means of light sealing and assay apparatus insertion and withdrawal. Thirdly it provides for a generally more accurate positioning of the sensitised surface of the strip over the detector.

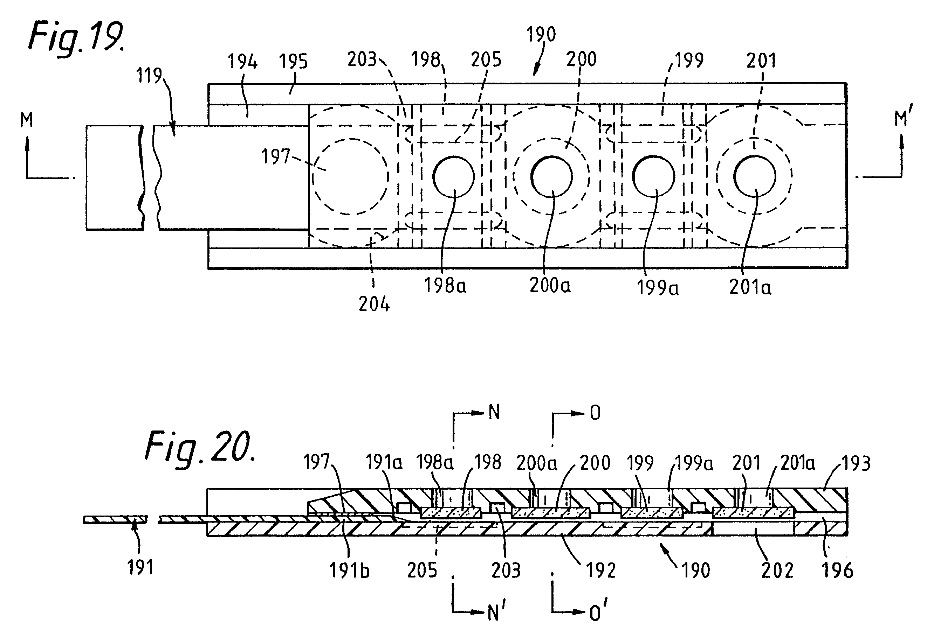

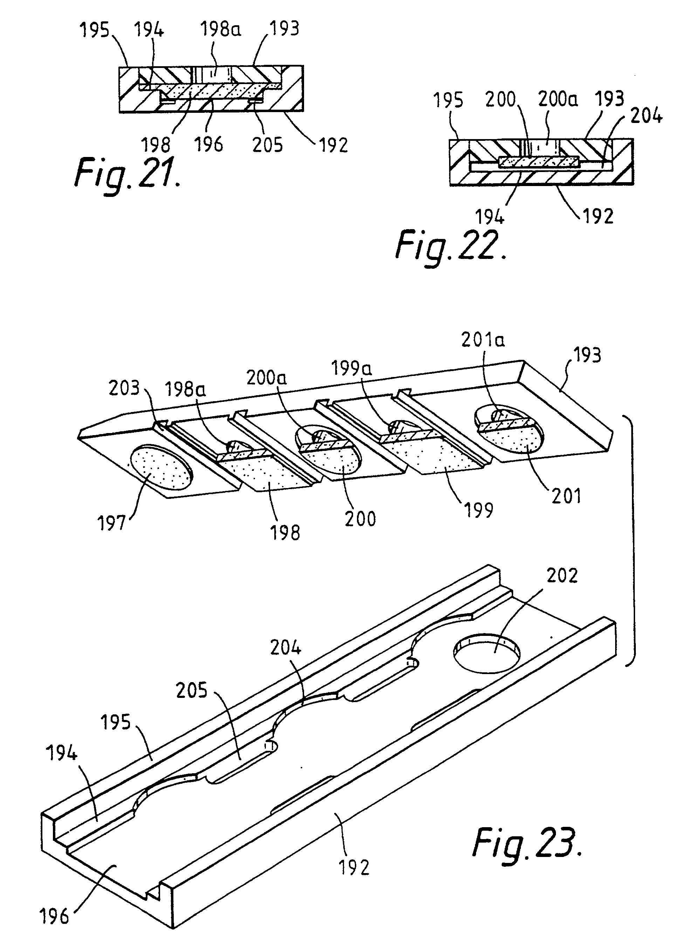

Referring to figs 19-23 these show a kit comprising a housing means shown overall 190 and a substrate in the form of a transparent elongate strip of plastic material 191 having a chambered leading edge 191a and an active surface portion 191b.

The housing means 190 is similar in overall construction to that shown in Figs 14 and 15 having a lower holder portion 192 and an upper holder portion 193. The lower holder portion 192 is provided with a longitudinally recessed base 194 and upwardly extending sidewalls 195 which together form a channel into which the upper holder portion 193 fits. In use the lower and upper holder portions 192 and 193 are held together by for example adhesive tape (not shown)

Lower holder portion 192 is further provided with a longitudinal recess 196 which when the upper holder portion 193 is in position as shown in Fig 19-23 forms a guide channel for the strip 191.

The upper holder portion 193 is provided with an absorbent sample transfer pad 197, first and second wiper pads 198, 199, which communicate with openings 198a, 199a respectively, to allow pads 198, 199 to be moistened and first and second reagent addition pads 200, 201, which communicate with reagent addition ports 200a, 201a respectively.

The lower holder portion 192 is provided with an parture 202 opposite second reagent addition pad 201. The position of aperture 201 allows any signal emitted from area 191b to pass through strip 191 to be detected outside the housing 190.

Sample and reagent addition pads 197, 200, 201 are less wide than the width of the strip 191, but wiper pads 198, 199 are wider than the width of strip 191 to ensure complete wiping of strip 191.

The upper holder portion 193 is provided with four transverse slots 203 to minimise cross-contamination between sample and reagents on the upper surface of strip 191. The recessed base 194 of the lower holder portion 192 is provided with six cut out portions 204 in the area about the sample and reagent transfer pads 197, 200, 201. These cut out portions minimise liquid being transferred longitudinally down the length of the apparatus by the capillary channel formed between the strip 191 and the channel formed by the recess 196. The lower holder portion 192 is also provided with four elongated grooves 205 in the area beneath the wiping pads 198, 199. These grooves 205 minimise wetting of the bottom of the strip 191 by liquid present on the wiping pads 198, 199.

The kit of Figs 19-23 may for example be used in a similar manner to the apparatus 100 as described above in a luminescence measuring device.

The effectiveness of the elements which comprise the first embodiment of the apparatus 1 will now be described:

## 1. EFFECTIVENESS OF WIPER PAD 4 IN REMOVING EXCESS SAMPLE FROM THE STRIP 8

This was studied using two different experiments.

### a) Horseradish peroxidase conjugate (chemiluminescent detection)

10 µL of a 1:1000 dilution of anti-human AFP - horseradish peroxidase conjugate (Dakopatts, Denmark) in phosphate buffered saline (Oxoid, UK) was applied to the sample pad 3. After a delay of 15 seconds the strip 8 was pushed through the holder 2. The pads 3, 4, 5, were then removed and the presence of conjugate determined using an enhanced chemiluminescent assay. Then individual pads were placed in wells of a microtitre plate and 150 µl of chemiluminescent substrate solution added (1.25 mmol/l luminol, 2.7 mmol/l hydrogen peroxide and 30 µmol/l p-hydroxycinnamic acid in 0.1 mol/l Tris buffer pH 8.6). The microtitre plate was placed in a luminometer and the intensity of light emission measured. The light emission (relative light units) from the different pads was as follows: sample pad (3) >20000; first wiper pad (4) 1492; first reagent pad (5) 7. This showed that the first wiper pad 4 effectively removed conjugate from the strip and virtually none was transferred to the adjacent reagent pad 5.

### b) Fluorescent dye

A similar experiment was performed using 10 µl of a dilute aqueous solution of fluorescein (5 mg/l; BDH Poole, UK). The dissassembled device was viewed under UV light which showed that the fluorescent dye had been removed by the leading edge of the first wiper pad 4 and virtually none had transferred to the first reagent pad 5.

## 2. EFFECTIVENESS OF WIPER PADS IN REMOVING EXCESS REAGENT FROM THE STRIP 8

This was studied using several different types of reagent:

### a) Coloured Dye

The apparatus 1 was assembled and 80 µl of a saturated aqueous solution of Procion Blue H-5R (BDH, Poole, UK) added to the first reagent pad 5. After a delay of 1 minute the strip 8 was pushed through the holder 2 until the end of the strip was beneath the optical window 13. The device 60 was disassembled and the various reagent and wiper pads 5, 6, 7 examined for the presence of the blue dye. The dye was only visible on the reagent pad 5 and on the leading edge of the wiper pad 6 adjacent to the reagent pad 5 indicating that the wiper pad 6 had efficiently removed the excess dye from the strip 8 and none had transferred to the second reagent pad 7.

### b) Fluorescent Dye

A similar experiment was performed using a dilute aqueous solution of fluorescein (5 mg/l BDH, Poole, UK). The dis-assembled apparatus 1 was viewed under UV light and the fluorescent dye was only visible on the reagent pad 5 and on the leading edge of the wiper pad 6.

### c) Horseradish Peroxidase Conjugate (Colorimetric Detection

80 µl of a 1:500 dilution of anti-human IgG - horseradish peroxidase conjugate (Dakopatts, Denmark) was applied to the first reagent pad 5 and after a delay of 30 minutes the strip 8 was pushed through the holder 2. The apparatus 1 was then dis-assembled and 80 µl of an o-phenylene diamine substrate solution added to the second wiper pad 6 and second reagent pad 7. After a delay of 30 minutes, inspection of the wiper pad 6 revealed the presence of conjugate on the leading edge (a yellow colour developed) and no evidence of conjugate on the reminder of the pad 6 or on any part of the second reagent pad 7.

## EFECTIVENESS OF REAGENT TRANSFER FROM THE REAGENT PADS 5 AND 7 TO THE SENSITISED AREA 14 OF THE STRIP 8 This was demonstrated using various reagents applied to the two reagent pads.

### a) Colorimetric Assay

A 10 x 10 mm area 14 at one end of a 75 x 10 mm clear polyvinyl chloride strip 8 was sensitised by contacting the one end with 20 µl of a 1:2 dilution of human IgG (50 g/l) in bicarbonate buffer (50 mmol/l, pH 9.6) spread on a glass plate. After 90 minutes the strip 8 was washed with PBS-Tween (100 mmol/l phosphate buffer pH 7.4 containing 150 mmol/l saline and 0.05% Tween 20). It was then pushed into the holder 2 such that the sensitised area 14 was under the first reagent pad 5. A 1:500 dilution of anti-human IgG - peroxidase conjugate (80 µl) in PBS was added to the reagent pad 5 and the strip 8 left to incubate for 30 minutes at room temperature. The strip 8 was then pushed further into the holder 2 past the wiper pad 6 until it rested directly under the second reagent pad 7. This was saturated with 80 µl of an orthophenylene diamine - substrate mixture (Abbott Laboratories, USA). The strip 8 was then left to incubate at room temperature for ten minutes with area 14 under pad 7. Inspection of the transparent strip 8 revealed the development of a dark yellow colour at the interface between reagent pad 7 and the sensitised area 14 of the strip 8 indicating the presence of bound conjugate.

### (b) Chemiluminescent Assay

A clear polyvinyl chloride strip 8 was sensitised by contacting a 10 x 10 mm area 14 of one end with 20 µl or a 1:2 dilution of human IgG in bicarbonate buffer (50 mmol/L pH 9.6) spread on glass plate. After incubation at room temperature for 2 hours the strip 8 was washed with PBS-Tween and then pushed into the holder 2 until the sensitised area 14 was in contact with the first reagent pad 5. A 1:500 dilution of antihuman IgG-peroxidase conjugate (80 µl) was added to the reagent pad 5 and the strip left to incubate for 30 minutes at room temperature. The strip was then pushed further into the holder 2 past the wiper pad 6 until it rested directly beneath the second reagent pad 7. The reagent pad 7 was saturated with 80 µl of chemiluminescent substrate solution (1.25 mmol/l luminol, 2.7 mmol/l hydrogen peroxide and 30 µmol/l p-hydroxycinnamic acid in 0.2 mol/l Tris buffer, pH 8.6). The apparatus 1 was then disassembled and strip 8 still in contact with the second reagent pad 7 transferred to a luminometer. Light emission from the interface between the sensitised area 14 of the strip 8 and the pad 7 was monitored through the clear strip. Light emission (in excess of the blank measurement) was detected thus indicating the presence of bound conjugate. Some light emission (four-fold less) was also detected when the strip-reagent pad assembly was monitored with the opaque pad facing the photodetector.

### Model Assay 1 Measurement of Mouse IgG

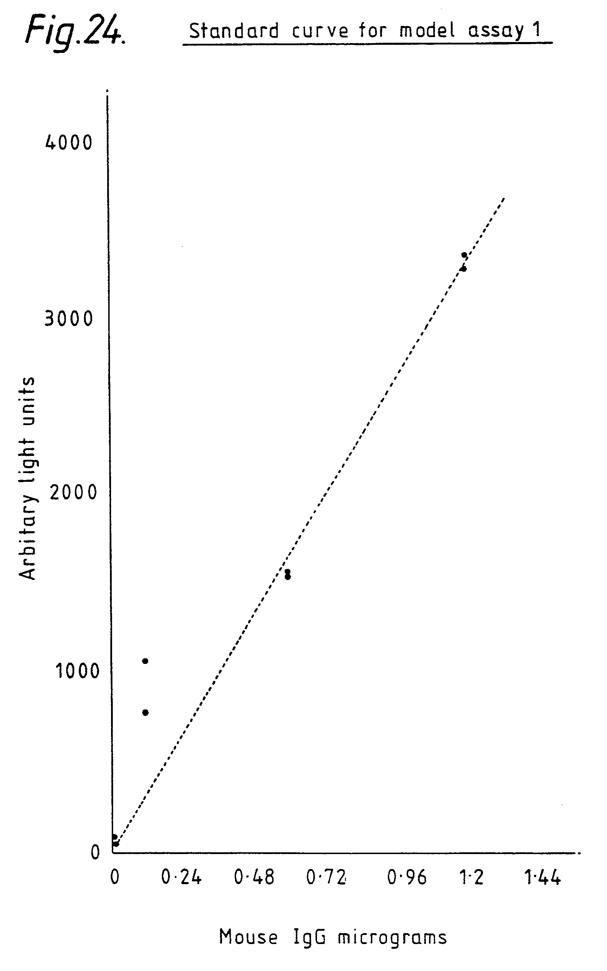

Clear ethanol-washed polyvinyl chloride strips 191 (60 x 10mm) were sensitised at one end 191 b over an area of 10 x 10mm by contacting the end with a solution of final concentration 10 µg/ml rabbit anti-mouse-immuogolbulin (Dakopatts, Denmark) diluted in 0.1M sodium carbonate buffer pH 9.6. After overnight incubation at 4°C the strips 191 were blotted dry before use. The strip was pushed into the holding means 190 such that the sensitised 191 b area was positioned under the sample pad 197. Samples (in a volume of 12 µl) were dilutions of mouse IgG (Sigma, Poole, Dorset) in phosphate-buffered saline plus 0.05% Tween 20 and 0.2% BSA (PSB/TB) and were added using a micropipette to sample pad 197. After 20 minutes incubation the strip was pushed past the first wiper pad 198 (which was wetted with 65 µl PBS added via port 198a) and positioned directly beneath the first reagent pad 200. A 1:1000 dilution of anti-mouse-immunolglobulin-peroxidase conjugate, 45 µl (Dakopatts, Denmark) diluted in PBS/TB was added to the first reagent pad 200 via port 200a, and incubated for 20 minutes. The strip 191 was pushed past the second wiper pad 199 (wetted with 65 µl PBS added via port 199a) and positioned directly under the second reagent pad 201. Luminescent reagent (45 µl: 1.25 mmol/L luminol, 2.7 mmol/L Hydrogen peroxide and 0.136 mmol/L p-iodophenol in 0.1M Tris buffer pH 8.6) was added to the second reagent pad via port 201a and the ligth output was measured through the optical window 202 60 seconds after initiation of the reaction, using the measuring apparatus (Fig. 17). A standard curve is shown in figure 24.

### Model Assay 2

A series of polyvinyl chloride strips 8 were sensitised at one end with 10 µl of dilutions of human IgG (50 g/l) in bicarbonate buffer (50 mmol/l, pH 9.6) by incubation for one hour at room temperature. The strips 8 were then washed with PBS-Tween. Strips 8 were then loaded into corresponding holders 2 such that the sensitised area 14 of each strip was directly beneath the first reagent pad 5 of each older and 80 µl of anti-human IgG-peroxidase conjugate added to the pad. After a 30 minute incubation time at room temperature each strip 8 was pushed past the second wiper pad 6 and each apparatus 1 then dis-assembled. Eac strip 8 was removed and placed in a cuvette containing 200 µl chemiluminescent substrate solution. The intensity of light emission from the sensitised area 14 of each strip 8 was monitored. A dose related relationship between light intensity and the amount of IgG used to sensitise each strip 8 was obtained. (Strips sentisised with 50, 10, and 1 g/l IgG produced light intensities of 80, 75, and 30 relative light units respectively).

## MODEL ASSAY 3: MEASUREMENT OF HUMAN CHORIONIC GONADOTROPHIN (HCG)

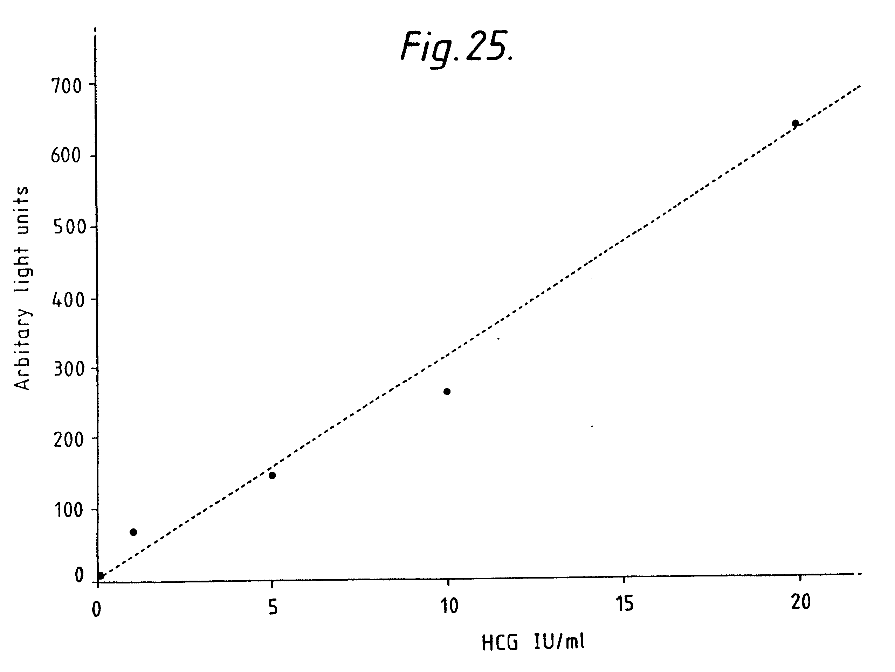

Clear polyvinyl chloride strips 191 (60 x 10 mm) were washed with 1% lipsol and wiped with ethanol. An area of 10 x 10 mm at one 191b end was coated with anti-HCG antibody (Serotec, UK) diluted to 10 µg/ml in 0.1 m carbonate buffer pH 9.6, by standing the strips 191 in the solution held in a glass vial (not shown) which had been precoated with 0,2% BSA in 0.1 m glycine. After overnight incubation at 4°C the strips 191 were soaked in 0.2% BSA in phosphate-buffered saline plus 0.05% tween (PBS/T) and then washed with PBS/T. The strips 191 were blotted dry. The strip was pushed into the channel 196 in the holder 190 such that the sensitised area 191b was positioned under the sample pad 197. Samples (in a volume of 12 µl) were dilutions of HCG (Sigma, Poole, Dorset) in PBS/T plus 0.2% BSA and were applied to the pad 197. After 20 minutes incubation with the sample, the strip 191 was pushed past the first wiper pad 198 which was wetted with 60 µl PBS added via port 198a; and positioned directly under the first reagent pad 200. A 1:500 dilution of a purpose made horseradish-peroxidase-anti-HCG conjugate (45 µl) was added to pad 109 via port 200a, and incubated for 20 minutes. The conjugate was made by linking anti-HCG (Dakopatts, Denmark) with type VI horseradish peroxidase (Sigma, Poole, Dorset). The conjugate was diluted in PBS/T + 0.2% BSA. The strip was then pushed past the second wiper pad 199 (wetted with 60 l PBS added via port 199a and positioned directly under the second reagent pad 201. Luminescent reagent (45 µl : 1.25 mmol/L Luminol, 2.7 mmol/L hydrogen peroxide and 0.136 mmol/L p-iodophenol in 0.1 M Tris buffer pH 8.6) was added to the second reagent pad via port 201a and the light output was measured through the optical window 202 60 seconds after initiation of the reaction, using the measuring apparatus (Fig 12). A standard curve is shown in figure 25.

## Claims

1. Assay apparatus for use in the detection and/or quantitative measurement of an analyte immobilised on an active surface portion of a solid substrate, characterised by:

a substrate housing means for retaining the solid substrate at one or more predetermined positions;

first reagent transfer means for transferring a solubilised first reagent to the surface portion when the substrate is held at one of the predetermined positions thereby coating the surface portion with solubilised reagent, the reagent being selected to react with either the analyte or the surface portion and emit a signal, either directly or in the presence of a signal stimulant, providing a measure of the amount of analyte immobilised on the surface portion, and:

substrate wiping means, the solid substrate and wiping means being moveable relative to each other when the substrate is wholly or partly retained within the housing means, said wiping means being arranged to make intimate wiping contact with all or part of the surface portion before and/or after transfer of solubilised first reagent thereto by the first reagent transfer means to remove excess liquid therefrom.

2. Assay apparatus as claimed in claim 1 characterised in that it includes at least one wiping means arranged to wipe the surface portion after transfer of first reagent.

3. Assay apparatus as claimed in Claim 2 characterised by having sample transfer means arranged to transfer sample solution to the surface portion of the substrate within the housing means.

4. Assay apparatus as claimed in claim 3 characterised in that the sample transfer means is arranged to transfer sample solution to the surface portion before the transfer of the first reagent.

5. Assay apparatus as claimed in claim 1, 2, 3 or 4 characterised by having substrate wiping means, the solid substrate and wiping means being moveable relative to each other when the substrate is wholly or partly retained within the housing means, said wiping means being arranged to make intimate wiping contact with all or part of the surface portion after transfer of sample solution and between the transfer of sample solution and first reagent.

6. Assay apparatus as claimed in claim 1, 2, 3 or 4 characterised by means for directing a signal stimulant onto the surface portion of the solid substrate.

7. Assay apparatus as claimed in claim 6 characterised in that the signal stimulant is particulate, electromagnetic radiation, heat, electrical, chemical or a combination thereof.

8. Assay apparatus as claimed in claim 7 characterised in that the signal stimulant is or includes a chemical stimulant and the apparatus includes second reagent transfer means arranged to transfer solubilised second reagent to the surface portion.

9. Assay apparatus as claimed in claim 1 or 2 characterised in that said wiping means are absorbent pads.

10. Assay apparatus as claimed in claim 5 characterised in that said wiping means is an abosrbent pad.

11. Assay apparatus as claimed in claim 1, 2, 3, 4 or 8 characterised in that at least one of said transfer means is an absorbent pad.

12. Assay apparatus as claimed in claim 11 characterised in that first and/or second reagent transfer means contain lyophilised reagent.

13. Assay apparatus as claimed in claim 1 characterised by having means for directing the emitted signal out of the housing means.

14. Assay apparatus as claimed in claim 1, 2 or 3 characterised by two or more cavities, each cavity containing wiping means or sample or reagent transfer means provided that at least one of each means is present in the apparatus, and each cavity having substrate guide means adapted to guide the substrate in the cavity.

15. Assay apparatus as claimed in claim 14 characterised in that the apparatus is further provided with means to disengage the wiping means from the surface of the substrate.

16. Assay apparatus as claimed in claim 1, 2 or 3 characterised in that the housing means comprises one or more cavities for receiving the substrate, each cavity containing transfer means, wiping means disposed in the opening of each cavity and substrate guide means for guiding the substrate into each cavity past the wiping means such that the wiping means makes wiping contact with the surface portion of the substrate.

17. Assay apparatus as claimed in claim 1, 2 or 3 characterised in that the housing means is adapted to house a flexible solid substrate in the form of an endless tape, and substrate guide means which include drive means for moving the tape past the transfer and wiping means.

18. Assay apparatus as claimed in claim 1, 2 or 3 characterised in that the housing means comprises an enclosure having a substrate guide means in the form of a channel therein, the transfer means and wiping means being linearly disposed along the channel in the path of the substrate.

19. Assay apparatus as claimed in claim 18 characterised in that the housing means contains wiping and transfer means arranged to perform the following sequence of steps:
(i) transfer of sample to substrate surface portion,
(ii) wiping of surface portion,
(iii) transfer of first reagent to surface portion,
(iv) wiping of surface portion,
(v) transfer of second reagent to surface portion,
(vi) emission and detection of emitted signal, and wherein steps (i) and/or (ii) are optional.

20. A Kit for detection and/or quantitative measurement of an analyte immobilised on an active surface portion of a solid substrate, characterised by comprising;

an assay apparatus as claimed in claim 1 and,

a solid substrate, capable of being housed within the housing means of the assay apparatus and having an active surface portion for immobilising thereon analyte present in a sample solution.

21. A kit as claimed in claim 20 characterised in that the substrate is a strip of plastic or derivatised plastic material.

22. A kit as claimed in claim 21 characterised in that the strip has a surface portion coated with a layer of antibodies, DNA or a chemical reagent.

23. A substrate adapted for use in a kit as claimed in any one of claims 20, 21 or 22.

24. Wiping or reagent transfer means in the form of an absorbent pad, adapted for use in a kit as claimed in any one of claims 20, 21 or 22.

25. A method of using the apparatus according to claim 1 comprising detecting and/or quantitatively measuring an analyte immobilised on a surface of a solid substrate caracterised by the steps of;
(a) positioning the surface of the substrate at a first predetermined position within a substrate housing means,
(b) coating the surface of the substrate at the first position with an amount of a solubilised first reagent that binds to the analyte, the amount of first reagent being in excess to that required for binding to all the immobilised analyte,
(c) advancing the substrate through the housing means to displace the surface from the first to a second predetermined position,
(d) wiping excess unbound reagent from the surface of the substrate as the substrate is advanced to second position, by contacting the said surface with a substrate wiping means mounted within the housing means,
(e) coating the surface of the substrate at the second position with an amount of a second solubilised reagent, that reacts with the first reagent to produce a detectable signal, the amount of second reagent being sufficient to react with all the bound first reagent, and
(f) detecting the emitted signal.

26. An analytical device comprising the assay apparatus according to claim 1 and a signal measuring device comprising photon or ionising particle detection means, detector housing means housing the detection means and adapted to receive the substrate housing means of the assay apparatus, detected signal amplification means, aplified signal processing means, data storage and/or display means, and locating means for locating the substrate housing means in the detector housing means at a signal receiving position such that the detection means is in signal recieving alignment with the signal emitted by the assay apparatus.

## Patentansprüche

1. Prüfvorrichtung zur Verwendung bei der Erfassung und/oder der quantitativen Messung eines auf einem aktiven Oberflächenbereich eines festen Substrates festgesetzten Analyts, gekennzeichnet durch:

eine Gehäuseeinrichtung für das Substrat, um das feste Substrat an einer oder mehreren vorbestimmten Positionen zu halten;

erste Reagenzienübertragungseinrichtungen, um ein erstes, gelöstes Reagenz an den Oberflächenbereich zu übertragen, wenn das Substrat an einer der vorbestimmten Positionen gehalten wird, um dadurch den Oberflächenbereich mit dem gelösten Reagenz zu überdecken, wobei das Reagenz ausgewählt ist, so daß es entweder mit dem Analyt oder dem Oberflächenbereich reagiert und entweder direkt oder in Anwesenheit eines Signalstimulatiums ein Signal emittiert, um so ein Maß für die Menge des auf dem Oberflächenbereich festgesetzten Analyts bereitzustellen und:

Substratwischeinrichtungen, wobei das feste Substrat und die Wischeinrichtungen relativ zueinander beweglich sind, wenn das Substrat ganz oder teilweise innerhalb der Gehäuseeinrichtung gehalten ist, wobei die Wischeinrichtungen dazu ausgelegt sind, mit dem gesamten oder einem Teil des Oberflächenbereichs einen engen Wischkontakt herzustellen, bevor und/oder nachdem durch die ersten Reagenzienübertragungseinrichtungen das gelöste erste Reagenz dorthin gebracht wurde, um überschüssige Flüssigkeit davon zu entfernen.

2. Prüfvorrichtung nach Anspruch 1, dadurch gekennzeichnet, daß sie zumindest eine Wischrichtung aufweist, die dazu ausgelegt ist, den Oberflächenbereich zu wischen, nachdem das erste Reagenz übertragen wurde.

3. Prüfvorrichtung nach Anspruch 2, dadurch gekennzeichnet, daß sie Probenübertragungseinrichtungen aufweist, die dazu ausgelegt sind, Probelösungen zum Oberflächenbereich des Substrats innerhalb der Gehäuseeinrichtung zu übertragen.

4. Prüfvorrichtung nach Anspruch 3, dadurch gekennzeichnet, daß die Probenübertragungseinrichtungen dazu ausgelegt sind, vor der Übertragung des ersten Reagenz Probelösungen zum Oberflächenbereich zu übertragen.

5. Prüfvorrichtung nach Anspruch 1, 2, 3 oder 4, gekennzeichnet durch Substratwischeinrichtungen, wobei das feste Substrat und die Wischeinrichtungen relativ zueinander beweglich sind, wenn das Substrat ganz oder teilweise innerhalb der Gehäuseeinrichtung zurückgehalten ist, wobei die Wischeinrichtungen dazu ausgelegt sind, mit dem gesamten oder einem Teil des Oberflächenbereichs einen engen Wischkontakt herzustellen, nachdem die Probelösung übertragen wurde, und zwischen dem Übertrag der Probelösung und dem des ersten Reagenz.

6. Prüfvorrichtung nach Anspruch 1, 2, 3 oder 4, gekennzeichnet durch Einrichtungen, die ein Signalstimulantium auf dem Oberflächenbereich des festen Substrats errichten.

7. Prüfvorrichtung nach Anspruch 6, dadurch gekennzeichnet, daß das Signalstimulantium ein aus Partikeln bestehender Stoff, elektromagnetische Strahlung, Hitze, elektrischer oder chemischer Natur, oder eine Kombination hiervon ist.

8. Prüfvorrichtung nach Anspruch 7, dadurch gekennzeichnet, daß das Signalstimulantium ein chemisches Stimulantium ist oder enthält und daß die Vorrichtung zweite Reagenzienübertragungseinrichtungen aufweist, um ein gelöstes zweites Reagenz zum Oberflächenbereich zu übertragen.

9. Prüfvorrichtung nach Anspruch 1 oder 2, dadurch gekennzeichnet, daß die Wischeinrichtungen absorbierende Bäusche sind.

10. Prüfvorrichtung nach Anspruch 5, dadurch gekennzeichnet, daß die Wischeinrichtungen ein absorbierender Bausch sind.

11. Prüfvorrichtung nach Anspruch 1, 2, 3, 4 oder 8, dadurch gekennzeichnet, daß zumindest eine der Übertragungseinrichtungen ein absorbierender Bausch ist.

12. Prüfvorrichtung nach Anspruch 11, dadurch gekennzeichnet, daß die ersten und/oder zweiten Übertragungseinrichtungen ein lyophiles Reagenz enthalten.

13. Prüfvorrichtung nach Anspruch 1, gekennzeichnet durch Einrichtungen, um das emittierte Signal aus der Gehäuseeinrichtung zu führen.

14. Prüfvorrichtung nach Anspruch 1, 2 oder 3, gekennzeichnet durch zwei oder mehr Aussparungen, wobei jede Aussparung Wischeinrichtungen oder Übertragungseinrichtungen für Reagenzien oder Proben aufweist, die so vorgesehen sind, daß zumindest eine dieser Einrichtungen in der Vorrichtung vorhanden sind, wobei jede Aussparung Substratführungseinrichtungen aufweist, die dazu ausgelegt sind, das Substrat in die Aussparung zu führen.

15. Prüfvorrichtung nach Anspruch 14, dadurch gekennzeichnet, daß die Vorrichtung außerdem mit Einrichtungen versehen ist, um die Wischeinrichtungen von der Substratoberfläche zu lösen.

16. Prüfvorrichtung nach Anspruch 1, 2 oder 3, dadurch gekennzeichnet, daß die Gehäuseeinrichtungen eine oder mehrere Aussparungen aufweisen, um das Substrat zu empfangen, wobei jede Aussparung Übertragungseinrichtungen beinhaltet, Wischeinrichtungen, die in der Öffnung jeder Aussparung vorgesehen sind, sowie Substratführungseinrichtungen, um das Substrat in jede der Aussparungen an den Wischeinrichtungen vorbeizuführen, so daß die Wischeinrichtungen mit dem Oberflächenbereich des Substrats einen wischenden Kontakt bilden.

17. Prüfvorrichtung nach Anspruch 1, 2 oder 3, dadurch gekennzeichnet, daß die Gehäuseeinrichtung dazu ausgelegt ist, ein festes, biegsames Substrat in Form eines Endlosbandes einzuschließen, und ebenso Substratführungseinrichtungen, die Antriebseinrichtungen zum Bewegen des Bandes hinter die Übertragungs- und Wischeinrichtungen aufweisen.

18. Prüfvorrichtung nach Anspruch 1, 2 oder 3, dadurch gekennzeichnet, daß die Gehäuseeinrichtungen einen Einschluß aufweisen, der eine Substratführungseinrichtung in Form eines darin ausgebildeten Kanals aufweist, wobei die Übertragungseinrichtungen und die Wischeinrichtungen linear längs des Kanal im Pfad des Substrats angeordnet sind.

19. Prüfvorrichtung nach Anspruch 18, dadurch gekennzeichnet, daß die Gehäuseeinrichtungen Wisch- und Übertragungseinrichtungen aufweisen, die dazu ausgelegt sind, die folgende Abfolge von Schritten durchzuführen:

(I) Übertragen einer Probe auf den Substratoberflächenbereich,

(II) Wischen des Oberflächenbereichs,

(III) Übertragen des ersten Reagenz zum Oberflächenbereich,

(IV) Wischen des Oberflächenbereichs,

(V) Übertragen des zweiten Reagenz zum Oberflächenbereich,

(VI) Aussenden und Erfassen des emittierten Signals, wobei die Schritte (I) und/oder (II) optional sind.

20. Ausrüstung zur Erfassung und/oder zur quantitativen Messung eines Analyts, das auf dem aktiven Oberflächenbereich eines festen Substrats festgesetzt ist, gekennzeichnet durch

eine Prüfvorrichtung nach Anspruch 1 und

ein festes Substrat, das innerhalb der Gehäuseeinrichtung der Prüfvorrichtung untergebracht werden kann und das einen aktiven Oberflächenbereich hat, um darauf ein in einer Probelösung vorhandenes Analyt festsetzen zu können.

21. Ausrüstung nach Anspruch 20, dadurch gekennzeichnet, daß das Substrat ein Plastikstreifen oder ein Abkömmling eines Plastikmaterials ist.

22. Ausrüstung nach Anspruch 21, dadurch gekennzeichnet, daß der Streifen einen Oberflächenbereich hat, der mit einer Schicht aus Antikörpern, DNA oder einem chemischen Reagenz beschichtet ist.

23. Ein Substrat, das zur Verwendung in einer Ausrüstung nach einem der Ansprüche 20, 21 oder 22 ausgelegt ist.

24. Wisch- oder Reagenzienübertragungseinrichtungen in Form eines absorbierenden Bausches, die zur Verwendung in einer Ausrüstung nach einem der Ansprüche 20, 21 oder 22 ausgelegt sind.

25. Verfahren zur Verwendung der Vorrichtung nach Anspruch 1 einschließlich des Erfassens und/oder der quantitativen Messung eines Analyts, das auf der Oberfläche eines festen Substrats festgesetzt wurde, gekennzeichnet durch die Schritte:

a) Positionieren der Oberfläche des Substrats an einer ersten vorbestimmten Position innerhalb einer Gehäuseeinrichtung für das Substrat,

b) Überdecken der Oberfläche des Substrats an der ersten Position mit einer Menge eines gelösten ersten Reagenz, das sich an das Analyt bindet, wobei die Menge des ersten Reagenz die Menge überschreitet, die zum Binden des gesamten festgesetzten Analyts benötigt wird,

c) Weiterbefördern des Substrats durch die Gehäuseeinrichtung, um die Oberfläche von der ersten zu einer zweiten vorbestimmten Position zu verschieben,

d) Abwischen von überschüssigem, ungebundenem Reagenz von der Oberfläche des Substrats, wenn das Substrat zur zweiten Position weiterbefördert wird, indem die Oberfläche mit einer Substratwischeinrichtung, die innerhalb der Gehäuseeinrichtung angebracht ist, in Berührung gebracht wird,

e) Überdecken der Oberfläche des Substrats an der zweiten Position mit einer Menge eines zweiten gelösten Reagenz, das mit dem ersten Reagenz reagiert, um ein erfaßbares Signal zu erzeugen, wobei die Menge des zweiten Reagenz ausreicht, um mit dem gesamten gebundenen ersten Reagenz zu reagieren, und

f) Erfassen des emittierten Signals.

26. Analysevorrichtung mit der Prüfvorrichtung nach Anspruch 1 und einer Signalmeßeinrichtung mit einer Erfassungseinrichtung für Photonen oder ionisierte Partikel, mit Detektorgehäuseeinrichtungen, die die Detektoreinrichtungen einschließen und die dazu ausgelegt sind, die Gehäuseeinrichtungen des Substrats der Prüfvorrichtung aufzunehmen, Verstärkungseinrichtungen für die empfangenen Signale, Verarbeitungseinrichtungen für die verstärkten Signale, Datenspeicher- und/oder -anzeigeeinrichtungen für die Daten, und Stelleinrichtungen, um die Gehäuseeinrichtungen für das Substrat in den Detektorgehäuseeinrichtungen in eine Signalempfangsposition zu stellen, so daß die Erfassungseinrichtungen in Signalempfangsausrichtung mit dem durch die Prüfvorrichtung emittierten Signal ist.

## Revendications

1. Appareil d'analyse destiné à être utilisé pour la détection et/ou la mesure quantitative d'un analyte immobilisé sur une partie de surface active d'un substrat solide, caractérisé par :

un boîtier de substrat destiné à retenir le substrat solide à une ou plusieurs positions prédéterminées,

un premier dispositif de transfert de réactif destiné à transférer un premier réactif solubilisé à la partie de surface lorsque le substrat est maintenu dans l'une des positions prédéterminées, avec revêtement de la partie de surface par le réactif solubilisé, le réactif étant choisi de manière qu'il réagisse soit avec l'analyte soit avec la partie de surface et émette un signal, soit directement soit en présence d'un stimulant de signaux, et donne ainsi une mesure de la quantité d'analyte immobilisée sur la partie de surface, et

un dispositif d'essuyage de substrat, le substrat solide et le dispositif d'essuyage étant mobiles l'un par rapport à l'autre lorsque le substrat est retenu partiellement ou totalement dans le boîtier, le dispositif d'essuyage étant destiné à être en contact intime par essuyage avec une partie ou la totalité de la partie de surface avant et/ou après le transfert du premier réactif solubilisé par le premier dispositif de transfert de réactif de manière que l'excès de liquide soit retiré.

2. Appareil d'analyse selon la revendication 1, caractérisé en ce qu'il comprend un dispositif d'essuyage destiné à essuyer la partie de surface après le transfert du premier réactif.

3. Appareil d'analyse selon la revendication 2, caractérisé en ce qu'il comporte un dispositif de transfert d'échantillon destiné à transférer une solution échantillon sur la partie de surface du substrat à l'intérieur du boîtier.

4. Appareil d'analyse selon la revendication 3, caractérisé en ce que le dispositif de transfert d'échantillon est destiné à transférer une solution échantillon à la partie de surface avant le transfert du premier réactif.

5. Appareil d'analyse selon la revendication 1, 2, 3 ou 4, caractérisé en ce qu'il comporte un dispositif d'essuyage de substrat, le substrat solide et le dispositif d'essuyage étant mobiles l'un par rapport à l'autre lorsque le substrat est retenu partiellement ou totalement dans le boîtier, le dispositif d'essuyage étant destiné à être en contact intime avec une partie ou la totalité de la partie de surface en en assurant l'essuyage après transfert de la solution échantillon et entre le transfert de la solution échantillon et celui du premier réactif.

6. Appareil d'analyse selon la revendication 1, 2, 3 ou 4, caractérisé par un dispositif destiné à diriger un stimulant de signaux sur la partie de surface du substrat solide.

7. Appareil d'analyse selon la revendication 6, caractérisé en ce que le stimulant de signaux est particulaire, sous forme d'un rayonnement électromagnétique, de chaleur, électrique, chimique ou une combinaison de tels stimulants.

8. Appareil d'analyse selon la revendication 7, caractérisé en ce que le stimulant de signaux est ou contient un stimulant chimique, et l'appareil contient un second dispositif de transfert de réactif destiné à transférer un second réactif solubilisé à la partie de surface.

9. Appareil d'analyse selon la revendication 1 ou 2, caractérisé en ce que le dispositif d'essuyage est formé de tampon absorbant.

10. Appareil d'analyse selon la revendication 5, caractérisé en ce que le dispositif d'essuyage est un tampon absorbant.

11. Appareil d'analyse selon la revendication 1, 2, 3, 4 ou 8, caractérisé en ce que l'un au moins des dispositifs de transfert est un tampon absorbant.

12. Appareil d'analyse selon la revendication 11, caractérisé en ce que le premier et/ou le second dispositif de transfert de réactif contient un réactif lyophilisé.

13. Appareil d'analyse selon la revendication 1, caractérisé en ce qu'il comporte un dispositif destiné à diriger le signal émis à l'extérieur du boîtier.

14. Appareil d'analyse selon la revendication 1, 2 ou 3, caractérisé par deux cavités ou plus, chaque cavité contenant un dispositif d'essuyage ou un dispositif de transfert d'échantillon ou un dispositif de transfert de réactif, pourvu que l'un au moins de chaque dispositif soit présent dans l'appareil, et chaque cavité a un dispositif de guidage de substrat destiné à guider le substrat dans la cavité.

15. Appareil d'analyse selon la revendication 14, caractérisé en ce que l'appareil comporte en outre un dispositif destiné à séparer le dispositif d'essuyage de la surface du substrat.

16. Appareil d'analyse selon la revendication 1, 2 ou 3, caractérisé en ce que le boîtier comporte une ou plusieurs cavités destinées à loger le substrat, chaque cavité contenant un dispositif de transfert, un dispositif d'essuyage placé dans l'ouverture de chaque cavité et un dispositif de guidage du substrat dans chaque cavité le long du dispositif d'essuyage afin que le dispositif d'essuyage soit en contact avec la partie de surface du substrat et l'essuie.

17. Appareil d'analyse selon la revendication 1, 2 ou 3, caractérisé en ce que le boîtier est destiné à loger un substrat solide flexible sous forme d'une bande sans fin, et un dispositif de guidage de substrat qui comporte un dispositif d'entraînement destiné à déplacer la bande le long des dispositifs de transfert et d'essuyage.

18. Appareil d'analyse selon la revendication 1, 2 ou 3, caractérisé en ce que le boîtier comporte une enceinte ayant un dispositif de guidage de substrat sous forme d'un canal placé à l'intérieur, le dispositif de transfert et le dispositif d'essuyage étant disposés linéairement le long du canal sur le trajet du substrat.

19. Appareil d'analyse selon la revendication 18, caractérisé en ce que le boîtier contient des dispositifs d'essuyage et de transfert destinés à effectuer la séquence suivante d'opérations :

(i) le transfert d'un échantillon à la partie de surface du substrat,

(ii) l'essuyage de la partie de surface,

(iii) le transfert du premier réactif à la partie de surface,

(iv) l'essuyage de la partie de surface,

(v) le transfert d'un second réactif à la partie de surface,

(vi) l'émission et la détection du signal émis, les étapes (i) et/ou (ii) étant éventuelles.

20. Kit de détection et/ou de mesure quantitative d'un analyte immobilisé sur une partie de surface active d'un substrat solide, caractérisé en ce qu'il comprend :

un appareil d'analyse selon la revendication 1, et

un substrat solide, capable d'être logé dans le boîtier de l'appareil d'analyse et ayant une partie de surface active destinée à immobiliser l'analyte présent dans une solution échantillon.

21. Kit selon la revendication 20, caractérisé en ce que le substrat est une bande de matière plastique ou de matière plastique dérivée.

22. Kit selon la revendication 21, caractérisé en ce que la bande a une partie de surface revêtue d'une couche d'anticorps, d'ADN ou d'un réactif chimique.

23. Substrat destiné à être utilisé dans un kit selon l'une quelconque des revendications 20, 21 et 22.

24. Dispositif d'essuyage ou de transfert de réactif sous forme d'un tampon absorbant, destiné à être utilisé dans un kit selon l'une quelconque des revendications 20, 21 et 22.

25. Procédé d'utilisation de l'appareil selon la revendication 1, comprenant la détection et/ou la mesure quantitative d'un analyte immobilisé sur une surface d'un substrat solide, caractérisé par les étapes suivantes :

(a) le positionnement de la surface du substrat à un premier emplacement prédéterminé dans un boîtier logeant le substrat,

(b) le revêtement de la surface du substrat, au premier emplacement, par une quantité d'un premier réactif solubilisé qui se fixe à l'analyte, la quantité du premier réactif étant supérieure à celle qui est nécessaire à la liaison de la totalité de l'analyte immobilisé,

(c) l'avance du substrat dans le boîtier afin que la surface soit déplacée du premier emplacement à un second emplacement prédéterminé,

(d) l'essuyage de l'excès de réactif non lié de la surface du substrat lorsque celui-ci avance vers une seconde position, par mise en contact de la surface avec un dispositif d'essuyage de substrat monté dans le boîtier,

(e) le revêtement de la surface du substrat, au second emplacement, d'une quantité d'un second réactif solubilisé qui réagit avec le premier réactif en donnant un signal détectable, la quantité du second réactif étant suffisante pour qu'il réagisse avec le totalité du premier réactif lié, et

(f) la détection du signal émis.

26. Dispositif analytique comprenant l'appareil d'analyse selon la revendication 1 et un dispositif de mesure de signaux comprenant un dispositif de détection de photons ou de particules ionisantes, un boîtier de détecteur logeant le dispositif de détection et destiné à contenir le boîtier du substrat de l'appareil d'analyse, un dispositif d'amplification du signal détecté, un dispositif de traitement du signal amplifié, un dispositif de mémorisation et/ou d'affichage de données, et un dispositif de positionnement du boîtier du substrat dans le boîtier du détecteur en position de réception de signaux de manière que le dispositif de détection soit aligné sur le signal émis par l'appareil d'analyse et puisse recevoir les signaux.

Fig.1.

Fig.2.

Fig.3.

Fig.4.

Fig.5.

Fig.6.

Fig.7. ∨

Fig.8.

Fig.9.

*Fig.10.*

*Fig.13.*

27

Fig.11.

Fig.12.

EP 0 357 625 B1

*Fig.14.*

*Fig.15.*

Fig.16.

103 108 116

105

105

102 104 101

146

156 148 120

100 150 138 134

130 136

158

K

142 162 144 156 126 148 122 128 124 134

K'

Fig.17.

146

142 144 146 158 152 154 114 100 138 150

130 136 L'

162 164

L

156 126 140 122 132 128 150 124

160 148 138

166 168

Fig.18.

120

EP 0 357 625 B1

Fig.19.

Fig.20.

Fig.21.

Fig.22.

Fig.23.

Fig.24.    Standard curve for model assay 1

Fig.25.

EP 0 357 625 B1